# EUROPEAN PATENT SPECIFICATION

(11) **EP 4 564 222 B1**
(45) Date of publication and mention of the grant of the patent: **15.07.2026**
(21) Application number: 23307103.4
(22) Date of filing: 30.11.2023
(51) Int. Cl.: G06K 13/00, A61B 50/00, B65B 3/00, G06K 17/00

(54) **SYSTEM AND METHOD FOR RFID-BASED LOCALIZATION OF MEDICAL INJECTION DEVICES IN A NEST**
SYSTEM UND VERFAHREN ZUR RFID-BASIERTEN LOKALISIERUNG VON MEDIZINISCHEN INJEKTIONSVORRICHTUNGEN IN EINEM NESTER
SYSTÈME ET PROCÉDÉ DE LOCALISATION BASÉE SUR RFID DE DISPOSITIFS D'INJECTION MÉDICAUX DANS UN NID

(43) Date of publication of application: 04.06.2025
(60) Divisional application: 26183392.5
(73) Proprietor: Becton Dickinson France, 38800 Le Pont-de-Claix (FR)
(72) Inventor: RIVIER, Cédric, 38340 Voreppe (FR); DOLEGA, Lukasz, 38950 St Martin Le Vinoux (FR); PETIT, Louis, 38610 Venon (FR); DURET, Enzo, 38000 Grenoble (FR); OZTURK, Senturk, 38130 Echirolles (FR)
(74) Representative: Germain Maureau

(56) References cited:
- AU-A1- 2020 299 964
- US-A1- 2008 035 234

## Description

### BACKGROUND OF THE INVENTION

### Field of the Invention

The present disclosure relates generally to RFID-tagged medical injection devices retained in packaging including a tub and nest and, more particularly, to a system and method for localizing RFID-tagged medical injection devices or components thereof within the nest via reading of an RFID tag on the nest.

### Description of Related Art

Documents US 2008/035234 A1 and AU 2020 299 964 A1 disclose systems for localizing RFID-tagged medical injection devices in a nest.

Medical injection devices, such as syringes, are used in a variety of environments for administering fluids, such as medications or other drugs, to a patient. Syringes may be provided as prefilled or pre-fillable syringes, which provide the convenience of rapidly delivering the liquid therein to a patient without the need to first aspirate the medication from another container and meter its volume. A syringe will typically include a syringe barrel having a distal end and a proximal end. A needle is connected at the distal end for injecting fluid into the patient and a plunger assembly is inserted through the proximal end that is movable within the barrel to force fluid out from the syringe through the needle.

Components of the syringes as described above often need to be transported from one site to another site during or after manufacturing. For instance, syringe barrels (which, as used herein, may be understood to include the barrel, the needle, and a needle cover) may be transported from a manufacturing site to a site at which the barrels may be filled with a "medicinal fluid," which can refer to a medication or another therapeutic agent used for treatment of chronic or acute conditions, as are known in the art. The filling of the syringe barrels is an automated process that is performed by a fill and finish machine, which dispenses fluid into the syringe barrels in an automated fashion.

During transportation and filling, the syringe barrels may be supported by a holding device, such as a tray or "nest." As known in the art, a nest can be a plate-shaped tray configured to support multiple medical containers in an upright orientation. Nests can comprise multiple "chimneys" aligned according to predetermined rows or a predetermined pattern, with each chimney defining a receptacle or opening configured to receive one medical container. When inserted into and through the receptacle, the medical container is held in the upright orientation in a direction substantially orthogonal to the nest. The nest is usually placed inside a box-shaped tub with an open top, which can be sealed by a sealing cover. The sealing cover can then be removed when the syringes are to be filled with a medicinal fluid by the fill and finish machine.

In an effort to automate the manufacturing of syringes (including filling of the syringes with medicinal fluid) and provide for further traceability of the syringes from the manufacturing process until the final labeling, the final use, or the disposal of the medical devices, it is becoming more common for such syringes to implement RFID tags (including a RFID chip and a RFID antenna) thereon that provide for remote identification and tracing of the syringes. As relates to the manufacturing process, RFID tags may be integrated into the syringes (e.g., in the needle shield thereof) at the site of initial manufacturing, with the tagged syringes (i.e., RFID-tagged syringe barrels) then loaded into a nest and tub for subsequent shipping. The location of each RFID-tagged syringe barrel within a nest (i.e., the specific chimney in which it is retained) may be recorded by the manufacturer and then provided to personnel at the location/facility at which filling of the syringe barrels is to be performed. Knowing an identifier of each syringe barrel and the location thereof within a nest, the fill and finish machine may be controlled to dispense an appropriate type and quantity of medicinal fluid into each syringe barrel retained within a nest.

While the above-described technique is typically efficient in performing an automated filling of RFID-tagged syringes via a fill and finish machine, it is recognized that errors may occur if a tub is mishandled. That is, it is possible for a tub to be loaded improperly onto a conveyor that advances the tub/nest downstream to a location of the fill and finish machine, with an example being that an orientation of the tub is flipped 180 degrees from its desired orientation - which may occur due to the symmetric profile of the tub and the inability of a person or loading system to determine if the tub is loaded in reverse (i.e., rotated 180 degrees) from its desired/proper orientation. When such improper loading of a tub occurs, the "known" ordering/location of the syringes within the nest is no longer accurate, which may potentially lead to an improper type and/or quantity of medicinal fluid being dispensed by the fill and finish machine into syringe barrels retained within the nest.

Accordingly, a need exists in the art for a system and method by which the orientation of a tub and nest may be determined as part of an automated fill and finish manufacturing process. The system and method would desirably be capable of localizing each RFID-tagged syringe barrel within the nest via the determination of the orientation of the tub and nest, with such determination being performed via reading of an RFID tag associated with the nest that retains the syringe barrels.

### SUMMARY OF THE INVENTION

Provided herein is a system for localizing RFID-tagged medical injection devices in a nest as defined in claim 1. The system includes a conveyor system configured to convey a plurality of tubs along a conveying path, each of the plurality of tubs including a nest therein having a nest RFID tag thereon, with the nest retaining a plurality of RFID-tagged medical injection devices therein. The system also includes a plurality of RFID coupling elements positioned at a reading location along the conveying path, with the plurality of RFID coupling elements including at least a first RFID coupling element provided at a first position of the reading location and a second RFID coupling element provided at a second position of the reading location. The system further includes a RFID reader operably connected with the plurality of RFID coupling elements and configured to perform a reading of the nest RFID tag upon a respective tub passing the plurality of RFID coupling elements at the reading location, wherein reading the nest RFID tag comprises reading a nest identifier from the nest RFID tag. The system still further includes at least one processor coupled to a memory and configured to determine an orientation of the nest based on a reading of the nest RFID tag by either the first RFID coupling element or the second RFID coupling element, the orientation comprising a first orientation or a second orientation. The at least one processor is further configured to query a database using the nest identifier and the determined orientation of the nest, the database including an electronic product code of each of the plurality of RFID-tagged medical injection devices within a respective nest and a location of each of the plurality of RFID-tagged medical injection devices within the respective nest with the nest in each of the first orientation and the second orientation. The at least one processor is still further configured to register the location of each of the plurality of RFID-tagged medical injection devices within the respective nest based on the query.

In accordance with some aspects of the disclosure, each nest comprises a support plate and a plurality of chimneys extending upwardly from the support plate, each of the plurality of chimneys configured to retain a RFID-tagged medical injection device therein, the plurality of chimneys arranged in a plurality of columns, with each of the plurality of columns comprising a plurality of seats.

In accordance with some aspects of the disclosure, the nest RFID tag is positioned on the support plate of the nest.

In accordance with some aspects of the disclosure, registering the location of each of the plurality of RFID-tagged medical injection devices comprises registering the column and seat of each of the plurality of RFID-tagged medical injection devices.

In accordance with some aspects of the disclosure, the first orientation is rotated 180 degrees from the second orientation.

In accordance with some aspects of the disclosure, the first RFID coupling element is positioned on a first side of a conveyor of the conveyor system and the second RFID coupling element is positioned on a second side of the conveyor opposite the first side, and wherein the first RFID coupling element receives a signal response from the nest RFID tag before the second RFID coupling element receives a signal response from the nest RFID tag when the nest is in the first orientation, and the second RFID coupling element receives a signal response from the nest RFID tag before the first RFID coupling element receives a signal response from the nest RFID tag when the nest is in the second orientation.

In accordance with some aspects of the disclosure, the first RFID coupling element is positioned on a first side of a conveyor of the conveyor system and the second RFID coupling element is positioned on a second side of the conveyor opposite the first side, and wherein the first RFID coupling element receives a stronger signal response from the nest RFID tag than the second RFID coupling element when the nest is in the first orientation and the second RFID coupling element receives a stronger signal response from the nest RFID tag than the first RFID coupling element when the nest is in the second orientation.

In accordance with some aspects of the disclosure, the system further includes at least one trigger sensor configured to detect when a respective tub is at the reading location. For each tub on the conveyor system, the at least one sensor is configured to transmit a trigger signal to the RFID reader to cause the RFID reader to read the nest RFID tag of the nest in the respective tub in response to detecting that the tub is at the reading location, with the RFID reader configured to read the nest RFID tag on the respective nest in response to receiving the trigger signal from the at least one sensor.

In accordance with some aspects of the disclosure, the system further includes a fill and finish machine positioned downstream of the reading location and configured to fill the plurality of RFID-tagged medical injection devices with a fluid. The at least one processor is configured to control the fill and finish machine to fill each of the plurality of RFID-tagged medical injection devices with fluid based on the registered location of each of the plurality of RFID-tagged medical injection devices within the respective nest and based on the electronic product code of each of the plurality of RFID-tagged medical injection devices.

In accordance with some aspects of the disclosure, the plurality of RFID-tagged medical injection devices comprise a plurality of syringe barrels.

Also provided herein is a method for localizing RFID-tagged medical injection devices in a nest as defined in claim 11. The method includes conveying each of a plurality of tubs, via a conveyor system, along a conveying path, each of the plurality of tubs including a nest therein having a nest RFID tag thereon, the nest retaining a plurality of RFID-tagged medical injection devices therein. The method also includes providing a RFID reading system at a position along the conveying path, the RFID reading system comprising a plurality of RFID coupling elements positioned at a reading location along the conveying path, the plurality of RFID coupling elements including at least a first RFID coupling element provided at a first position of the reading location and a second RFID coupling element provided at a second position of the reading location, and a RFID reader operably connected with the plurality of RFID coupling elements. The method further includes performing a reading of each of nest RFID tag via the RFID reading system, as the respective tubs pass the reading location and, based on the reading of each of the plurality of nest RFID tags, determining, via at least one processor, a location of each of the plurality of RFID-tagged medical injection devices within a respective nest. In determining the location of each of the plurality of RFID-tagged medical injection devices, the method further comprises determining an orientation of the nest based on a reading of the nest RFID tag by either the first RFID coupling element or the second RFID coupling element, the orientation comprising a first orientation or a second orientation, querying a database using the nest identifier and the determined orientation of the nest, the database including an electronic product code of each of the plurality of RFID-tagged medical injection devices within a respective nest and a location of each of the plurality of RFID-tagged medical injection devices within a respective nest with the nest in each of the first orientation and the second orientation, and registering the location of each of the plurality of RFID-tagged medical injection devices within the respective nest based on the query.

In accordance with some aspects of the disclosure, each nest comprises a plurality of chimneys each configured to retain a RFID-tagged medical injection device therein, the plurality of chimneys arranged in a plurality of columns, with each of the plurality of columns comprising a plurality of seats, and wherein registering the location of each of the plurality of RFID-tagged medical injection devices comprises registering the column and seat of each of the plurality of RFID-tagged medical injection devices.

In accordance with some aspects of the disclosure, performing a reading of each of nest RFID tag via the RFID reading system comprises receiving a signal response from the nest RFID tag at the first RFID coupling element before receiving the signal response at the second RFID coupling element when the nest is in the first orientation or receiving a signal response from the nest RFID tag at the second RFID coupling element before receiving the signal response at the first RFID coupling element when the nest is in the second orientation.

In accordance with some aspects of the disclosure, performing a reading of each of nest RFID tag via the RFID reading system comprises receiving a signal response from the nest RFID tag at the first RFID coupling element that is stronger than a signal response received at the second RFID coupling element when the nest is in the first orientation or receiving a signal response from the nest RFID tag at the second RFID coupling element that is stronger than a signal response received at the first RFID coupling element when the nest is in the second orientation.

In accordance with some aspects of the disclosure, the method further includes detecting when a respective tub is at the reading location using at least one trigger sensor and, in response to detecting that the tub is at the reading location, transmitting a trigger signal from the at least one trigger sensor to the RFID reader to cause the RFID reader to read the nest RFID tag of a respective nest contained within the tub.

In accordance with some aspects of the disclosure, the method further includes controlling a fill and finish machine to fill each of the plurality of RFID-tagged medical injection devices retained in a respective nest with fluid based on the registered location of each of the plurality of RFID-tagged medical injection devices within the nest and based on the electronic product code of each of the plurality of RFID-tagged medical injection devices retained in the nest.

In accordance with some aspects of the disclosure, the first orientation is rotated 180 degrees from the second orientation.

### BRIEF DESCRIPTION OF THE DRAWINGS

FIG. 1A is a perspective view of a RFID-tagged syringe, with which embodiments of the disclosure may be implemented;
FIG. 1B is an exploded view of the syringe of FIG. 1A;
FIG. 1C is a side view of a needle shield included in the syringe of FIG. 1A;
FIG. 2 is a perspective view of packaging, including a tub and nest, for storing and shipping a plurality of medical components, such as the syringe of FIGS. 1A and 1B, with which embodiments of the disclosure may be implemented;
FIG. 3A is a top view of the nest of FIG. 2 in a first orientation;
FIG. 3B is a top view of the nest of FIG. 2 in a second orientation;
FIG. 4 illustrates an environment in which a method of reading a plurality of RFID-tagged nests, such as the nest of FIGS. 3A and 3B, may be performed, according to a non-limiting embodiment described herein;
FIG. 5 is a diagram of non-limiting embodiments or aspects of components of one or more devices and/or one or more systems of FIG. 4; and
FIG. 6 is a flowchart of a process for localizing RFID-tagged medical injection devices in a nest, according to a non-limiting embodiment described herein.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the scope of the present invention as defined in the appended claims.

In the present disclosure, the distal end of a component or of a device means the end furthest away from the hand of the user and the proximal end means the end closest to the hand of the user, when the component or device is in the use position, i.e., when the user is holding a syringe in preparation for or during use. Similarly, in this application, the terms "in the distal direction" and "distally" mean in the direction toward the distal tip of the syringe, and the terms "in the proximal direction" and "proximally" mean in the direction opposite the direction of the distal tip of the syringe.

As used herein, the terms "communication" and "communicate" may refer to the reception, receipt, transmission, transfer, provision, and/or the like of information (e.g., data, signals, messages, instructions, commands, and/or the like). For one unit (e.g., a device, a system, a component of a device or system, combinations thereof, and/or the like) to be in communication with another unit means that the one unit is able to directly or indirectly receive information from and/or transmit information to the other unit. This may refer to a direct or indirect connection that is wired and/or wireless in nature. Additionally, two units may be in communication with each other even though the information transmitted may be modified, processed, relayed, and/or routed between the first and second unit. For example, a first unit may be in communication with a second unit even though the first unit passively receives information and does not actively transmit information to the second unit. As another example, a first unit may be in communication with a second unit if at least one intermediary unit (e.g., a third unit located between the first unit and the second unit) processes information received from the first unit and communicates the processed information to the second unit. In some non-limiting embodiments or aspects, a message may refer to a network packet (e.g., a data packet and/or the like) that includes data. It will be appreciated that numerous other arrangements are possible.

As used herein, the term "computing device" may refer to one or more electronic devices that are configured to directly or indirectly communicate with or over one or more networks. A computing device may be a mobile or portable computing device, a desktop computer, a server, and/or the like that is specifically configured to provide one or more of the features and/or functions described herein. Furthermore, the term "computer" may refer to a specifically configured computing device that includes the necessary components to receive, process, and output data according to aspects described herein, and normally includes a display, a processor, a memory, an input device, and a network interface. A "computing system" may include one or more computing devices or computers. An "application" or "application program interface" (API) refers to computer code or other data sorted on a computer-readable medium that may be executed by a processor to facilitate the interaction between software components, such as a client-side front-end and/or server-side back-end for receiving data from the client. An "interface" refers to a generated display, such as one or more graphical user interfaces (GUIs) with which a user may interact, either directly or indirectly (e.g., through a keyboard, mouse, touchscreen, etc.). Further, multiple computers, e.g., servers, or other computerized devices directly or indirectly communicating in the network environment may constitute a "system" or a "computing system".

As used herein, the term "communication network" may refer to one or more wired and/or wireless networks. For example, a communication network may include a cellular network (e.g., a long-term evolution (LTE) network, a third generation (3G) network, a fourth generation (4G) network, a fifth generation (5G) network, a code division multiple access (CDMA) network, etc.), a public land mobile network (PLMN), a local area network (LAN), a wide area network (WAN), a metropolitan area network (MAN), a telephone network (e.g., the public switched telephone network (PSTN)), a private network, an ad hoc network, an intranet, the Internet, a fiber optic-based network, a cloud computing network, and/or the like, and/or a combination of these or other types of networks.

It will be apparent that systems and/or methods, described herein, can be implemented in different forms of hardware, firmware, or a combination of hardware and software. The actual specialized control hardware or software code used to implement these systems and/or methods is not limiting of the implementations. Thus, the operation and behavior of the systems and/or methods are described herein without reference to specific software code, it being understood that software and hardware can be designed to implement the systems and/or methods based on the description herein.

No aspect, component, element, structure, act, step, function, instruction, and/or the like used herein should be construed as critical or essential unless explicitly described as such. Also, as used herein, the articles "a" and "an" are intended to include one or more items, and may be used interchangeably with "one or more" and "at least one." Furthermore, as used herein, the term "set" is intended to include one or more items (e.g., related items, unrelated items, a combination of related and unrelated items, etc.) and may be used interchangeably with "one or more" or "at least one." Where only one item is intended, the term "one" or similar language is used. Also, as used herein, the terms "has," "have," "having," or the like are intended to be open-ended terms. Further, the phrase "based on" is intended to mean "based at least partially on" unless explicitly stated otherwise.

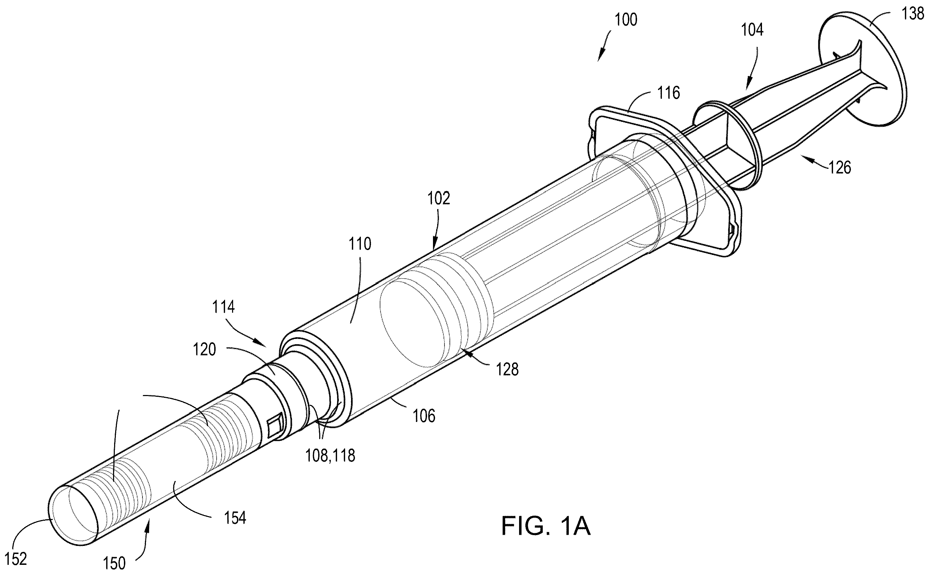

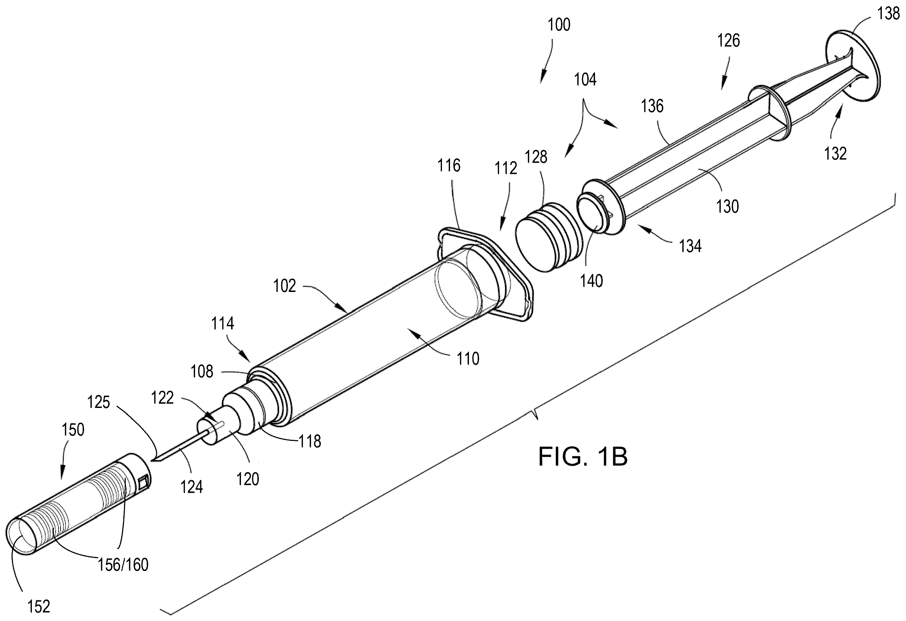

Referring to FIGS. 1A and 1B, shown is a non-limiting embodiment of a RFID-tagged medical injection device 10 with which aspects or embodiments of the disclosure may be implemented. While the medical device is shown and described hereafter as a syringe ("syringe 100"), it is recognized that other RFID-tagged medical devices, including other medical injection devices (e.g., auto-injectors) may be utilized with the system and method of the disclosure described in detail here below.

As shown in FIGS. 1A and 1B, the syringe 100 generally includes a syringe barrel assembly 102 (hereafter "syringe barrel 102") and a plunger assembly 104. The plunger assembly 104 is movable within the syringe barrel 102 along a longitudinal axis to an advanced position to facilitate administering of an injectable fluid (e.g., medication) to a patient, for example. The syringe barrel 102 is formed of a generally cylindrical outer wall 106 and an end member 108 that collectively define a chamber 110 for retaining fluid therein. The syringe barrel 102 includes an open proximal end 112 configured to receive the plunger assembly 104 therein and a distal end 114 at which end member 108 is positioned. The proximal end 112 of the syringe barrel 102 may include a flange 116 to facilitate handling and positioning of the syringe 100 and to maintain the relative position of the syringe barrel 102 with respect to the plunger assembly 104 during medication administration. At the distal end 114, the end member 108 may include a shoulder 118 which narrows with respect to the cylindrical outer wall 106, as well as a hub portion 120 extending out distally from shoulder 118. The hub portion 120 is formed as a partially hollow member that defines a channel 122 therethrough in fluid communication with the chamber 110. A needle 124 is attached to the hub portion 120 within channel 122, such as by being glued or otherwise secured to the hub portion 120, with the needle 124 extending distally from the hub 120 out to a distal needle tip 125.

The plunger assembly 104 of syringe 100 is formed of an elongate plunger rod 126 (more generally "plunger 126," as used hereafter) and a plunger head or stopper 128. The plunger 126 may include a main body 130 extending between a plunger proximal end 132 and a plunger distal end 134. In some embodiments, the main body 130 may include a plurality of elongate vanes or walls 136 extending axially along a length thereof between the plunger proximal end 132 and the plunger distal end 134. A thumb press 138 is positioned at the plunger proximal end 132 that may be engaged by a thumb (or other finger) of the user to apply a distally directed force to the plunger assembly 104 to move the plunger 126 with respect to the syringe barrel 102. In some embodiments, a flanged extension member 140 (e.g., disc-shaped flange) is positioned at the plunger distal end 134 that is configured to mate with the stopper 128. In other embodiments, the plunger distal end 134 may include a female receptacle formed therein that is configured to receive and connect to a protrusion (e.g., pin) extending out proximally from the stopper, with the protrusion and receptacle engaging via threading, for example.

As shown in FIGS. 1A and 1B, a needle shield 150 of syringe barrel 102 (i.e., of the syringe barrel assembly may be coupled to the hub portion 120 and/or shoulder 118 to protect the needle 124. According to aspects of the disclosure, the needle shield 150 may be composed of a rigid outer casing 152 that provides structure to the needle shield 150 and a compliant inner casing or "jupe" 154 that surrounds the needle 124 (when needle shield 150 is coupled to syringe barrel 102). In various embodiments, the outer casing 152 may be made of a rigid material such as polypropylene (PP), polyethylene (PE), polyethylene terephthalate (PET), polystyrene (PS) or polycarbonate (PC), while the inner casing 154 is made of a deformable material such as rubber (e.g., butyl rubber, isoprene rubber, latex rubber, silicone rubber) or a thermoplastic elastomer (TPE), as non-limiting examples.

According to aspects and embodiments of the disclosure, the needle shield 150 may include an RFID tag 156 integrated therein that allows for identification and/or tracking of the syringe 100. That is, the RFID tag 156 may include or store information thereon regarding the contents of the syringe 100 (i.e., the medication or drug included therein, if a prefilled syringe) and the manufacturing history of the syringe and/or may provide location information to an associated reader, so as to enable tracking of the syringe 100. Accordingly, the RFID tag 156 is considered to generally include thereon an electronic product code (EPC) that is specific to the syringe 100. According to aspects or embodiments of the disclosure, the RFID tag 156 may be integrated into the needle shield 150, such as by being inlaid or otherwise disposed between the outer casing 152 and the inner casing 154 of the needle shield 150. According to other embodiments, the RFID tag 156 may be applied to an outer surface of the needle shield 150. In each of these embodiments, the RFID tag 156 conforms to the surface(s) or wall(s) to or within which it is disposed, such that when the RFID tag 156 is integrated with/on a cylindrical component such as the needle shield 150, the RFID tag 156 will have a curvature that matches that of the needle shield 150.

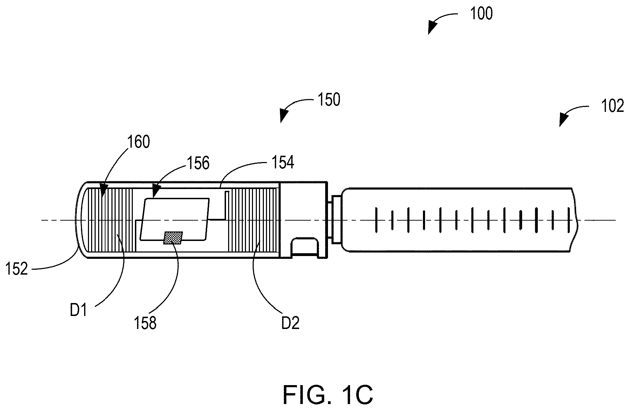

According to embodiments, and as shown in FIG. 1C, the RFID tag 156 includes an RFID chip 158 connected to an RFID dipole coupling element or antenna 160. The antenna 160 of RFID tag 156 may include two legs or dipole elements D1, D2, with the RFID chip 158 disposed between extremities of the poles D1, D2, such as being centered therebetween. In some embodiments, the RFID tag 156 may be positioned and oriented within needle shield 150 such that a dipole axis, A_{D}, of the RFID tag 156 is approximately aligned with a longitudinal axis, A_{C}, of the needle shield 150 (and a longitudinal axis, A_{S}, of the syringe 100), such that more strict quality controls may be implemented during manufacturing/assembly of the syringe 100 and the cap 150 thereof.

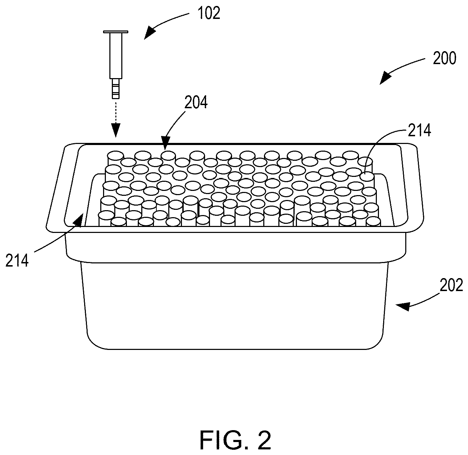

Referring to FIG. 2, shown is a non-limiting embodiment of packaging 200 that may be used for the packaging and transporting of a plurality of medical components therein, such as syringes 100 or syringe barrel assemblies 102 thereof shown in FIGS. 1A-1C. The packaging 200 includes a container or tub 202 in which a nest 204 may be housed, with the nest 204 configured to retain a plurality of syringes 100 or syringe barrels 102 therein. The packaging 200 may also include a sealing cover (not shown) that may be applied over the tub 202 after the nest 204 is packaged therein, to provide a sterile environment for storing and shipping the syringes 100 or syringe barrels 102.

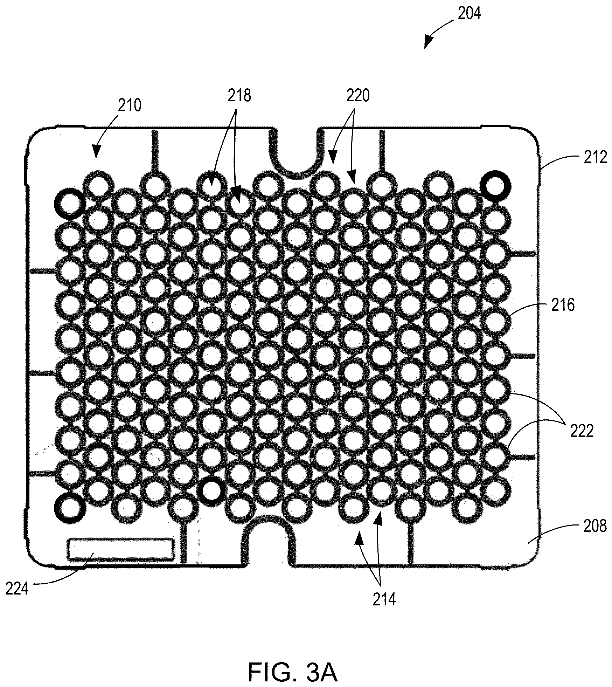

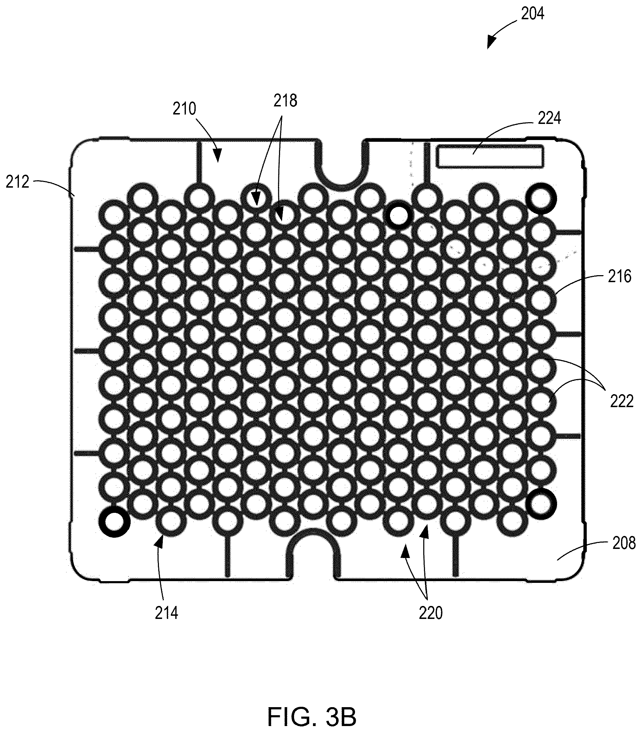

As shown in FIG. 2 and also in FIGS. 3A and 3B, an exemplary nest 204 that may be retained in tub 202 comprises a support plate 208 having a first or upper surface 210, a second or lower surface (now shown), and a peripheral edge 212 extending about the support plate 208. In some examples, the perimeter of support plate 208 is substantially rectangular in shape, while in other embodiments, the support plate 208 may be square. The nest 204 furthers comprise tubular walls, members, or ridges - referred to hereafter as "chimneys 214" - that protrude outward and upward from a plane defined by the upper surface 210 of the support plate 208. Each chimney 214 can include a cylindrical wall 216 that defines a receptacle 218 configured to receive a syringe barrel 102 therein. The receptacle 218 includes a top opening at a top end of the chimney 214 and a bottom opening at a bottom end of the chimney 214, with the receptacle sized to receive the cylindrical outer wall 106 of barrel 102 therein and the flange 116 of barrel 102 resting against an upper edge of the chimney 214. As shown best in FIGS. 3A and 3B, according to some aspects of the disclosure, the nest 204 can be configured to include one-hundred sixty (160) chimneys 214 for receiving syringe barrels 102, although it is recognized that another number of chimneys 214 may be provided on nest 204 that is dependent on the size of the syringe barrels 102 retained therein (e.g., 1-3 mL syringe barrels, 5 mL syringe barrels, 10 mL syringe barrels, etc.). The chimneys 214 may be considered as being arranged in a plurality of columns 220 (e.g., 16 columns, in the embodiment of FIGS. 3A and 3B), with each of the columns 220 including a plurality of "seats" 222 that correspond to the individual chimneys 214 (e.g., 10 seats in each column, with 160 seats total, in the embodiment of FIGS. 3A and 3B).

According to aspects of the disclosure, nest 204 includes an RFID tag 224 thereon, such as on the upper surface 210 of support plate 208, adjacent a corner thereof. The RFID tag 224 allows for identification of the nest 204. That is, the RFID tag 224 may include or store thereon a unique identifier (UID) that is specific to the nest 204, with it recognized that each specific nest 204 will have associated therewith the particular RFID-tagged syringe barrels 102 that are retained in the nest 204 and the location of each syringe barrel within the nest 204, so as to enable localizing of the syringe barrels 102 within nest 204. The RFID tag identifier of the nest 204 and of the RFID-tagged syringe barrels 102 retained thereby may be used for registration purposes and dispensing of a medication or drug into the syringe barrels 102 (if a prefilled syringe) during a fill and finish process, as explained in further detail below.

It is recognized that, depending on the orientation of the tub 202 and the nest 204 retained therein, the location of the RFID tag 224 and the location of columns 220 and individual seats 222 (of chimneys 214) may vary - with a first orientation of nest 204 shown in FIG. 3A and a second orientation of nest 204 shown in FIG. 3B. With regard to the RFID tag 224, the location of the RFID tag 224 may thus vary relative to a RFID reading system implemented to read the RFID tag 224. With regard to the location of the columns 220 and individual seats 222 (of chimneys 214), the location of particular RFID-tagged syringe barrels 102 that are retained in the nest 204 (i.e., the column 220 and seat 222 in which an RFID-tagged syringe barrel 102 is registered as being retained in) may vary (relative to a conveyor and/or fill and finish machine used to dispense medicinal fluid into the syringe barrels) depending on the orientation of the tub 202 and the nest 204 therein. For example, a RFID-tagged syringe barrel 102 identified as "syringe barrel #160" may be at a first seat location with the nest 204 in a first orientation (FIG. 3A) and at a different second seat location with the nest 204 in a second orientation (FIG. 3B).

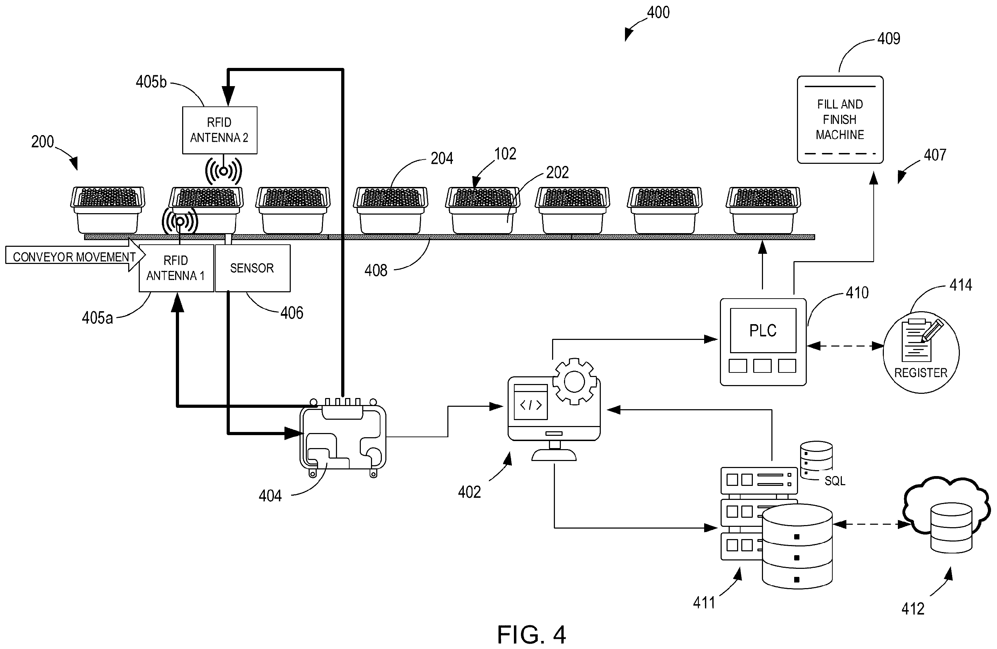

Referring now to FIG. 4, a manufacturing and reading environment or system 400 is illustrated within which or by which RFID-tagged nests of a packaging (such as the RFID-tagged nests 204 of packaging 200 of FIG. 2 and FIGS. 3A and 3B) may be read, in accordance with an aspect of the disclosure. As shown in FIG. 4, a plurality of packagings 200 (each including a tub 202 and nest 204) may be provided to system 400, which may be configured as a fill and finish manufacturing line. According to aspects of the disclosure, reading of the RFID-tagged nests 204 may be implemented as part of a process for localizing RFID-tagged syringe barrels 102 retained within each nest 204, including identifying the specific location (i.e., chimney 214) of each RFID-tagged syringe barrel 102 within the nest 204, as determined by an orientation of a tub 202 and nest 204 when provided to system 400. As indicated above, "syringe barrels 102" may be understood to refer to an assembly of the cylindrical outer wall 106, end member 108, shoulder 118, hub portion 120, needle 124, and needle shield 150 (including RFID tag 156).

As shown in FIG. 4 system 400 may include a controller system 402, a RFID reader 404, RFID coupling elements 405 including at least a first RFID coupling element 405a and a second RFID coupling element 405b (collectively a "RFID reading system"), a trigger sensor 406, a conveyor system 407 - including a conveyor 408, fill and finish machine 409, and programmable logic controller (PLC) 410 - a local database system 411, and/or an external database system 412.

Controller system 402 may include one or more devices capable of receiving information and/or data from RFID reader 404, trigger sensor 406, conveyor system 407, local database system 411, and/or external database system 412 (e.g., via a communication network, via a direct wired and/or wireless connection, etc.) and/or communicating information and/or data to RFID reader 404, trigger sensor 406, conveyor system 407, local database system 411, and/or external database system 412 (e.g., via communication network, via a direct wired and/or wireless connection, etc.). For example, controller system 402 may include a computing device, such as a server, a group of servers, and/or other like devices. In some non-limiting embodiments or aspects, controller system 402 may include middleware, such as Message Queuing Telemetry Transport (MQTT) protocolbased middleware, and/or the like, configured to manage communication with and/or control of operations of RFID reader 404, trigger sensor 406, conveyor system 407 (i.e., PLC 410 of conveyor system 407, etc.), local database system 411, and/or external database system 412.

RFID reader 404 may include one or more devices capable of receiving information and/or data from controller system 402, trigger sensor 406, conveyor system 407, local database system 411 and/or external database system 412 (e.g., via a communication network, via a direct wired and/or wireless connection, etc.) and/or communicating information and/or data to controller system 402, trigger sensor 406, conveyor system 407, local database system 411 and/or external database system 412 (e.g., via communication network, via a direct wired and/or wireless connection, etc.).

RFID reader 404 may include a passive RFID reader, an active RFID reader, or any combination thereof. RFID reader 404 may be configured to read - from the plurality of RFID tags 224 on the nests 204 (within tubs 202) in the production line - identifiers associated with the nests 204. For example, RFID reader 404 may be configured to attempt to read an RFID tag 224 on a nest 204 in response to receiving a trigger signal from trigger sensor 406.

RFID coupling elements 405a, 405b (which may be provided as near field antennas or other near field coupling elements, but are referred to hereafter generally as "antennas 405a, 405b") of RFID reader 404 may be located proximate to conveyor 408 such that, as conveyor 408 moves the tubs 202 along the production line, the tubs 202 are moved one-by-one to be adjacent to (and/or to be paused adjacent to for a predetermined period of time) antennas 405a, 405b of RFID reader 404 at a "reading location", and then moved past antennas 405a, 405b of RFID reader 404. While only a first antenna 405a and a second antenna 405b are shown in FIG. 4, it is recognized that system 400 could include one or more additional antennas, such that a plurality of antennas 405 (i.e., antennas 405a, 405b... 405n) are associated with the RFID reader 404. The antennas 405a, 405b are positioned at various locations along the conveyor 408 at the reading location (e.g., on opposing sides of the conveyor 408) to facilitate reading of the RFID tag 224 on each nest 204 in the event that that a tub 202 (and nest 204 therein) is oriented on conveyor 408 incorrectly. That is, it is recognized that a tub 202 may be loaded onto conveyor 408 in an orientation that is reversed from the correct orientation (i.e., rotated 180 degrees from the correct orientation) - this being due to the symmetry of the tub and the possibility of a loading device (or worker) loading the tub 202 on the conveyor 208 incorrectly - with the result being that the RFID tag 224 on the nest 204 (within tub 202) may be located adjacent either of opposing sides of the conveyor 408. Positioning of the antennas 405a, 405b at various locations (such as on opposing sides) of the conveyor 408 at the reading location thus ensures reading of the RFID tag 224 on each nest 204 regardless of the tub 202/nest 204 being arranged in a first orientation (FIG. 3A) or a second orientation (FIG. 3B) that is rotated 180 degrees from the first orientation.

Trigger sensor 406 may include one or more devices capable of receiving information and/or data from controller system 402, RFID reader 404, conveyor system 407, local database system 411 and/or external database system 412 (e.g., via a communication network, via a direct wired and/or wireless connection, etc.) and/or communicating information and/or data to controller system 402, RFID reader 404, conveyor system 407, local database system 411 (e.g., via communication network, via a direct wired and/or wireless connection, etc.).

Trigger sensor 406 may include at least one sensor (e.g., an optical sensor, a laser sensor, etc.) configured to detect or be trigged when an individual tub 202 is in a predetermined location relative to antennas 405a, 405b of the RFID reader 404 (e.g., when a tub 202 is at the reading location, adjacent antennas 405a, 405b). Trigger sensor 406 may be located proximate or adjacent to antennas 405a, 405b of RFID reader 404 and/or conveyor 408 and/or at a position relative to antennas 405a, 405b of RFID reader 404 and/or conveyor 408 at which, when a tub 202 is in the predetermined location relative to antennas 405a, 405b of the RFID reader 404, the tub 202 is within the field of detection or view of trigger sensor 406 such that trigger sensor 406 is triggered or detects the tub 202. In response to being triggered or detecting a tub 202 in the predetermined location relative to antennas 405a, 405b of the RFID reader 404, trigger sensor 406 may be configured to communicate or transmit a trigger signal to RFID reader 404 to trigger or cause RFID reader 404 to attempt to read a RFID tag on the nest 204 within tub 202.

Conveyor system 407 may include one or more devices capable of receiving information and/or data from controller system 402, RFID reader 404, trigger sensor 406, local database system 411 and/or external database system 412 (e.g., via a communication network, via a direct wired and/or wireless connection, etc.) and/or communicating information and/or data to controller system 402, RFID reader 404, trigger sensor 406, local database system 411 and/or external database system 412 (e.g., via communication network, via a direct wired and/or wireless connection, etc.).

Conveyor system 407 may include a conveyor 408 (e.g., a motorized belt conveyor, a motorized roller conveyor, a motorized chain conveyor, a motorized trolley conveyor, etc.) that conveys tubs 202 along a path (e.g., linear path). Each of the tubs 202 may be retained on the conveyor 408, with the tubs 202 held in position by mechanical force or another suitable means. Conveyor 408 may be configured to individually move (e.g., move one-by-one, etc.) tubs 202 in the production line adjacent to and past antennas 405a, 405b of RFID reader 404. For example, tubs 202 may be arranged on conveyor 408, and/or conveyor 408 may be configured to move tubs 202 together in a same direction such that tubs 202 are moved one-by-one adjacent to (and/or paused adjacent to for a predetermined period of time), and moved then past, antennas 405a, 405b of RFID reader 404.

The PLC 410 of conveyor system 407 is configured to control one or more operations of conveyor system 407, including conveyor 408 and/or fill and finish machine 409. The PLC 410 may be configured to track a sequential order of the tubs 202 in the production line (on conveyor 408) and the location of RFID-tagged syringe barrels 102 in the nests 204 of tubs 202, with such tracking performed via one or more shift registers (e.g., shift register 414) and/or sensors, according to existing manufacturing techniques. The PLC 410 may be configured to use the tracked order to control operation of the fill and finish machine 409 for dispensing medicinal fluid into the syringe barrels 102 of each nest 204, as described herein in more detail below.

The fill and finish machine 409 of conveyor system 207 is located downstream of a reading location on conveyor 408. The fill and finish machine 409 is configured to dispense medicinal fluid into the syringe barrels 102 retained within each nest 204, and thus may include a plurality of nozzles or dispensers for spraying/dispensing fluid out therefrom and into syringe barrels 102. In some embodiments, fill and finish machine 409 may be further configured to stopper the syringe barrels 102 retained within each nest 204 upon the filling thereof with medicinal fluid. The fill and finish machine 409 may use a known technique to introduce/insert a stopper (and plunger rod) into the syringe barrels 102, including vent tube stoppering or other suitable techniques. A fully assembled syringe may thus be provided after processing (i.e., filling and stoppering) performed by the fill and finish machine 409.

Local database system 411 may include one or more devices capable of receiving information and/or data from controller system 402, RFID reader 404, trigger sensor 406, conveyor system 407, and/or external database system 412 (e.g., via a communication network, via a direct wired and/or wireless connection, etc.) and/or communicating information and/or data to controller system 402, RFID reader 404, trigger sensor 406, conveyor system 407, and/or external database system 412 (e.g., via communication network, via a direct wired and/or wireless connection, etc.). In some non-limiting embodiments or aspects, local database system 411 includes one or more local databases (e.g., local to and/or implemented by controller system 402, etc.). Local database system 411 may be configured to store data on each RFID-tagged nest 204 (i.e., the UID of each nest RFID tag 224), along with data on the RFID-tagged syringe barrels 102 retained in each nest 204 and the ordering/locations of the RFID-tagged syringe barrels 102 retained in each nest 204, as explained in further detail below. In some embodiments, local database system 411 may retrieve data on each RFID-tagged nest 204 and data on the RFID-tagged syringe barrels 102 retained in each nest 204 from external database system 412, for subsequent storage on local database system 411.

External database system 412 may include one or more devices capable of receiving information and/or data from controller system 402, RFID reader 404, trigger sensor 406, conveyor system 407, and/or local database system 411 (e.g., via a communication network, via a direct wired and/or wireless connection, etc.) and/or communicating information and/or data to controller system 402, RFID reader 404, trigger sensor 406, conveyor system 407, and/or local database system 410 (e.g., via communication network, via a direct wired and/or wireless connection, etc.). In some non-limiting embodiments or aspects, external database system 412 includes one or more external databases (e.g., external to and/or not implemented by controller system 102, etc.). External database system 412 may be configured to store data on each RFID-tagged nest 204 (i.e., the UID of each nest RFID tag 224), along with data on the RFID-tagged syringe barrels 102 retained in each nest 204 and the ordering/locations of the RFID-tagged syringe barrels 102 retained in each nest 204, as explained in further detail below.

The number and arrangement of devices shown in FIG. 4 is provided as an example. There can be additional devices, fewer devices, different devices, or differently arranged devices than those shown in FIG. 4. Furthermore, two or more devices shown in FIG. 4 can be implemented within a single device, or a single device shown in FIG. 4 can be implemented as multiple, distributed devices. Additionally, or alternatively, a set of devices (e.g., one or more devices, etc.) of system 400 can perform one or more functions described as being performed by another set of devices of system 400.

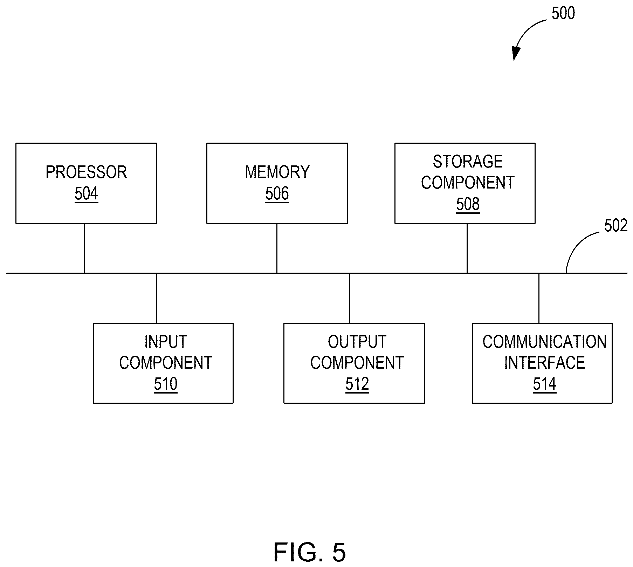

Referring now to FIG. 5, FIG. 5 is a diagram of example components of a device 500. Device 500 may correspond to one or more devices of controller system 402, RFID reader 404 (e.g., one or more devices of a system of RFID reader 404, etc.), trigger sensor 406 (e.g., one or more devices of a system of trigger sensor 406, etc.), one or more devices of conveyor system 407, one or more devices of local database system 411 and/or one or more devices of external database system 412. In some non-limiting embodiments or aspects, one or more devices of controller system 402, RFID reader 404 (e.g., one or more devices of a system of RFID reader 404, etc.), trigger sensor 406 (e.g., one or more devices of a system of trigger sensor 406, etc.), one or more devices of conveyor system 407 one or more devices of local database system 411 and/or one or more devices of external database system 412, may include at least one device 500 and/or at least one component of device 500. As shown in FIG. 5, device 500 may include bus 502, processor 504, memory 506, storage component 508, input component 510, output component 512, and communication interface 514.

Bus 502 may include a component that permits communication among the components of device 500. In some non-limiting embodiments or aspects, processor 504 may be specifically configured to perform one or more of the mixing aspects described in hardware, software, or a combination of hardware and software. For example, processor 504 may include a processor (e.g., a central processing unit (CPU), a graphics processing unit (GPU), an accelerated processing unit (APU), etc.), a microprocessor, a digital signal processor (DSP), and/or similar processing component (e.g., a field-programmable gate array (FPGA), an application-specific integrated circuit (ASIC), etc.) that can be programmed to perform a function. Memory 506 may include random access memory (RAM), read-only memory (ROM), and/or another type of dynamic or static storage device (e.g., flash memory, magnetic memory, optical memory, etc.) that stores information and/or instructions for use by processor 504.

Storage component 508 may store information and/or software related to the operation and use of device 500. For example, storage component 508 may include a hard disk (e.g., a magnetic disk, an optical disk, a magneto-optic disk, a solid state disk, etc.), a compact disc (CD), a digital versatile disc (DVD), a floppy disk, a cartridge, a magnetic tape, and/or another type of computer-readable medium, along with a corresponding drive.

Input component 510 may include a component that permits device 500 to receive information, such as via user input (e.g., a touch screen display, a keyboard, a keypad, a mouse, a button, a switch, a microphone, etc.). Additionally or alternatively, input component 510 may include a sensor for sensing information (e.g., a global positioning system (GPS) component, an accelerometer, a gyroscope, an actuator, etc.). Output component 512 may include a component that provides output information from device 500 (e.g., a display, a speaker, one or more lightemitting diodes (LEDs), etc.).

Communication interface 514 may include a transceiver-like component (e.g., a transceiver, a separate receiver and transmitter, etc.) that enables device 500 to communicate with other devices, such as via a wired connection, a wireless connection, or a combination of wired and wireless connections. Communication interface 514 may permit device 500 to receive information from another device and/or provide information to another device. For example, communication interface 514 may include an Ethernet interface, an optical interface, a coaxial interface, an infrared interface, a radio frequency (RF) interface, a universal serial bus (USB) interface, a Wi-Fi^{®} interface, a cellular network interface, and/or the like.

Device 500 may perform one or more processes described herein. Device 500 may perform these processes based on processor 504 (e.g., a central processing unit (CPU), a graphics processing unit (GPU), etc.) executing software instructions stored by a computer-readable medium, such as memory 506 and/or storage component 508. A computer-readable medium (e.g., a non-transitory computer-readable medium) is defined herein as a non-transitory memory device. A non-transitory memory device includes memory space located inside of a single physical storage device or memory space spread across multiple physical storage devices.

Software instructions may be read into memory 506 and/or storage component 508 from another computer-readable medium or from another device via communication interface 514. When executed, software instructions stored in memory 506 and/or storage component 508 may cause processor 504 to perform one or more processes described herein. Additionally or alternatively, hardwired circuitry may be used in place of or in combination with software instructions to perform one or more processes described herein.

Memory 506 and/or storage component 508 may include data storage or one or more data structures (e.g., a database, etc.). Device 500 may be capable of receiving information from, storing information in, communicating information to, or searching information stored in the data storage or one or more data structures in memory 506 and/or storage component 508.

The number and arrangement of components shown in FIG. 5 are provided as an example. In some non-limiting embodiments or aspects, device 500 may include additional components, fewer components, different components, or differently arranged components than those shown in FIG. 5. Additionally or alternatively, a set of components (e.g., one or more components) of device 500 may perform one or more functions described as being performed by another set of components of device 500.

According to aspects of the disclosure, it is recognized that reading of the RFID tags 224 of the nests 204 by the RFID reader 404 can be used to localize individual RFID-tagged syringe barrels 102 within each nest 204. Specifically, reading of the RFID tag 224 of each nest 204 can be used to determine an orientation of that nest 204 (and associated tub 202), as loaded onto conveyor 408 of system 400, with it recognized that tubs 202 may be inadvertently loaded onto conveyor 408 in a reverse orientation that is 180 degrees from desired. The orientation of a nest 204 (and tub 202) can be determined based on the receipt of a backscattered signal response from the RFID tag 224 on nest 204 by the first antenna 405a and second antenna 405b - including an order in which the signal response(s) are received and/or a strength of the signal response(s) that are received. The orientation of the nest 204 can then be utilized in order to properly localize and locate each RFID-tagged syringe barrel 102 retained within the nest 204, in order to properly register the RFID-tagged syringe barrels 102 and facilitate filling and finishing thereof (via machine 409).

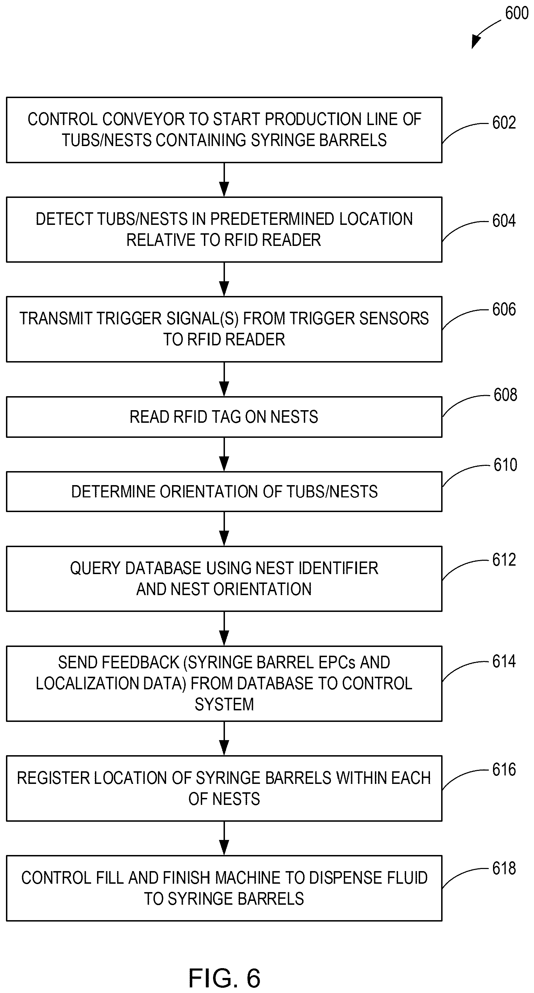

Referring now to FIG. 6, a flowchart is provided for a non-limiting embodiment or aspect of a method 600 for localizing individual RFID-tagged syringe barrels 102 within a nest 204 of a tub 202 that is advanced along a conveyor to a fill and finish machine. Specifically, method 600 reads the RFID tag of each nest to identify the nest and determine an orientation of the nest (and tub) relative to the conveyor and the fill and finish machine, with the location of each of the plurality of RFID-tagged medical injection devices within the respective nest being registered/recorded based on the nest identifier and the orientation of the nest.

In some non-limiting embodiments or aspects, one or more of the steps of process 600 may be performed (e.g., completely, partially, etc.) by controller system 402 (e.g., one or more devices of controller system 402, etc.). In some non-limiting embodiments or aspects, one or more of the steps of process 600 may be performed (e.g., completely, partially, etc.) by another device or a group of devices separate from or including controller system 402, such as RFID reader 404 (e.g., one or more devices of a system of RFID reader 404, etc.), trigger sensor 406 (e.g., one or more devices of a system of trigger sensor 406, etc.), conveyor system 407 (e.g., PLC 410), local database system 411 and/or external database system 412.

As shown in FIG. 6, at step 602, process 600 includes controlling a conveyor to start a production line of packages - i.e., of tubs/nests and syringe barrels retained therein. For example, controller system 402 may control conveyor system 407 to cause conveyor 408 to start conveyor movement to move the tubs 202/nests 204 in the production line adjacent to and past antennas 405a, 405b of RFID reader 404, i.e., to the reading location. As an example, controller system 402 (e.g., middleware of controller system 402, etc.) may send a signal to conveyor system 407 (e.g., the PLC 410 of conveyor system 407, etc.) to cause conveyor system 407 to control conveyor 408 to start conveyor movement. In such an example, controller system 402 (e.g., middleware of controller system 402, etc.) may send a signal to arm the RFID reader 404.

In moving the tubs 202/nests 204 along conveyor 408, it is recognized that the tubs 202/nests 204 in the production line may be in a first orientation or a second orientation. That is, it is recognized that a tub 202/nest may be loaded onto conveyor 408 in an orientation that is reversed from the correct orientation (i.e., rotated 180 degrees from the correct orientation) - this being due to the symmetry of the tub and the possibility of a loading device (or worker) loading the tub 202 on the conveyor 208 incorrectly - with the result being that the RFID tag 224 on the nest 204 (within tub 202) may be located adjacent either of opposing sides of the conveyor 408.

As shown in FIG. 6, at step 604, process 600 includes detecting a tub in a predetermined location relative to an antenna of a RFID reader. For example, for each tub/nest of the plurality of tubs 202/nests 204 in the production line, trigger sensor 406 may detect when that tub 202/nest 204 is in a predetermined location relative to antennas 405a, 405b of RFID reader 404 (i.e., at the reading location).

As shown in FIG. 6, at step 606, process 600 includes transmitting a trigger signal to a RFID reader. For example, for each tub/nest of the plurality of tubs 202/nests 204 in the production line, trigger sensor 406 may, in response to detecting that the tub 202/nest 204 is at a predetermined location relative to antennas 405a, 405b of RFID reader 404 in the production line (i.e., at the reading location), transmit a trigger signal to RFID reader 404 to trigger or cause RFID reader 404 to read the nest RFID tag 224 of the nest 204.

As shown in FIG. 6, at step 608, process 600 includes reading a nest RFID tag on a nest. For example, for each tub/nest of the plurality of tubs 202/nests 204 in the production line, RFID reader 404 may, in response to receiving the trigger signal from trigger sensor 406 (e.g., corresponding to that tub/nest, etc.), read the RFID tag 224 on that nest 204. As an example, RFID reader 404 may transmit radio waves via antennas 405a, 405b (and any other additional antennas, if more are including in system 400) to RFID tag 224. According to aspects of the disclosure, the antennas 405a, 405b are positioned at various locations along the conveyor 408 at the reading location, such as on opposing sides of the conveyor 408. When at the reading location, radio waves from the first antenna 405a and the second antenna 405b will activate the RFID tag 224. Responsive to receiving the radio waves, the nest RFID tag 224 may transmit a (backscattered) signal that includes an identifier associated with that nest 204, back to the first antenna 405a and the second antenna 405b, to be read by RFID reader 404. According to embodiments of the disclosure, the timing and/or strength of the backscattered signals received by the first antenna 405a and the second antenna 405b will vary depending on whether the tub 202/nest 204 is arranged in a first orientation (with RFID tag 224 proximate to first antenna 405a) or a second orientation (with RFID tag 224 proximate to second antenna 405b), as explained further below.

The RFID reader 404 may read, from the RFID tags 224 on nests 204 in the production line, a plurality of identifiers (e.g., a plurality of UIDs, etc.) associated with the plurality of nests 204 (e.g. as the plurality of tubs 202/nests 204 in the production line are individually moved adjacent to antennas 405a, 405b of RFID reader 404 by conveyor 408, etc.). In such an example, for each nest of the plurality of nests 204 in the production line, RFID reader 404 may transmit or stream, to controller system 402 (e.g., to middleware of controller system 402, etc.) the information or data read from the RFID tag 224 (e.g., the identifier, etc.).

In addition to the RFID reader 404 transmitting information or data read from the RFID tags 224 (e.g., the identifier, etc.) to controller system 402, the RFID reader 404 also transmits information or data to controller system 402 regarding the orientation of each of the tubs 202/nests 204. That is, the RFID reader 404 also transmits information or data to controller system 402 regarding the order and/or strength of backscatter signals from the RFID tag 224 of each nest 204 that were received by the first antenna 405a or the second antenna 405b - with the reception of backscatter signals received by the first antenna 405a and the second antenna 405b dependent on whether a tub 202/nest 204 was positioned in a first orientation or a second orientation on conveyor 408.

In one embodiment, the RFID reader 404 transmits information or data to controller system 402 regarding the order in which backscatter signals from the RFID tag 224 of each nest 204 were received by the first antenna 405a and the second antenna 405b. With a tub 202/nest 204 positioned on conveyor 408 in a first orientation, the first antenna 405a will receive a signal response from RFID tag 224 before a signal response from RFID tag 224 is received by the second antenna 405b. Conversely, with a tub 202/nest 204 positioned on conveyor 408 in a second orientation, the second antenna 405b will receive a signal response from RFID tag 224 before a signal response from RFID tag 224 is received by the first antenna 405a.

In another embodiment, the RFID reader 404 transmits information or data to controller system 402 regarding the strength of backscatter signals from the RFID tag 224 of each nest 204 received by the first antenna 405a and the second antenna 405b. With a tub 202/nest 204 positioned on conveyor 408 in a first orientation, the first antenna 405a will receive a signal response from RFID tag 224 that is stronger/higher than a signal response from RFID tag 224 received by the second antenna 405b. Conversely, with a tub 202/nest 204 positioned on conveyor 408 in a second orientation, the second antenna 405b will receive a signal response from RFID tag 224 that is stronger/higher than a signal response from RFID tag 224 received by the first antenna 405a.

While the receipt of a signal response from a nest RFID tag 224 by only a first antenna 405b and a second antenna 405b in system 400 is described above, it is recognized that method 600 also covers embodiments where a plurality of antennas 405 (i.e., more than two antennas 405) are included in system 400. In such an embodiment, the order and/or strength of backscatter signals from the RFID tag 224 of each nest 204 received by the plurality of antennas is analyzed/compared in order to determine the orientation of the tub 202/nest 204.

As shown in FIG. 6, at step 610, process 600 includes determining whether each tub 202/nest 204 is positioned in a first orientation or a second orientation on conveyor 408. As described above, information or data transmitted from the RFID reader 404 to controller system 402 indicates the order and/or strength of backscatter signals from the RFID tag 224 of each nest 204 that were received by the first antenna 405a and the second antenna 405b, and controller system 402 may analyze such information/data to determine the orientation of each tub 202/nest 204.

As shown in FIG. 6, at step 612, process 600 includes - for each nest - querying a database using a nest identifier and a determined orientation of the nest. That is, for each nest RFID tag 224 read by RFID reader 404, controller system 402 (e.g., middleware of controller system 402, etc.) may immediately query local database system 411, which includes data stored therein on an EPC of each of the plurality of RFID-tagged syringe barrels 102 retained within a respective nest 204 (as identified from a UID stored on a nest RFID tag 224) and a location of each of the plurality of RFID-tagged syringe barrels 102 within the respective nest 224 - with locations of the plurality of RFID-tagged syringe barrels 102 within a nest 204 being stored for the nest 204 in each of the first orientation and the second orientation.

In some embodiments, the data stored in local database system 411, including the EPCs of the RFID-tagged syringe barrels 102 retained within a respective nest 204 and a location of each of the plurality of RFID-tagged syringe barrels 102 within the respective nest 204, is periodically synchronized with external database system 412. The external database system 412 may, for example, include the EPCs of the RFID-tagged syringe barrels 102 retained within a respective nest 204 and a location of each of the plurality of RFID-tagged syringe barrels 102 within the respective nest 204 (as identified by a UID stored on the RFID tag 224 thereof), with such data provide provided by the syringe barrel manufacturer.

Responsive to the querying of local database system 411 at step 612, process 600 continues at step 614, where the local database system 411 transmits feedback to the controller system 402 that includes a listing of the EPCs of the RFID-tagged syringe barrels 102 retained within a respective nest 204 and a location of each of the plurality of RFID-tagged syringe barrels 102 within the respective nest 204. The feedback provided to controller system 402 regarding the location of each of the plurality of RFID-tagged syringe barrels 102 within the respective nest 204 is specific to the determined orientation of that nest 204 (and tub 202), such that a first listing of locations of RFID-tagged syringe barrels 102 within the respective nest 204 is provided back to controller system 402 if the nest 204 is in the first orientation and a second listing of locations of RFID-tagged syringe barrels 102 within the respective nest 204 is provided back to controller system 402 if the nest 204 is in the second orientation.

As shown in FIG. 6, subsequent to receiving feedback from a local database system at step 614, the process 400 continues at step 616. At step 616, in response to receiving feedback from the local database system, a controller system operably communicates with a PLC of a conveyor system and registers the location of each of the plurality of RFID-tagged medical injection devices within a respective nest. For example, in response to receiving feedback from the local database system 411, controller system 402 operably communicates with the PLC 410 of conveyor system 407 and registers the location of each of the plurality of RFID-tagged syringe barrels 102 within each respective nest 204. The registered location of each of the plurality of RFID-tagged syringe barrels 102 will take into account the determined orientation of the nest 204 (and tub 202) on conveyor 408, in order to correctly locate each syringe barrel 102.

As shown in FIG. 6, at step 618, process 600 includes controlling a fill and finish machine to fill each of the plurality of RFID-tagged medical injection devices retained in a respective nest with fluid based on the registered location of each of the plurality of RFID-tagged medical injection devices within the nest and based on the EPC of each of the plurality of RFID-tagged medical injection devices retained in the nest. For example, with the EPC and the location of each of the plurality of RFID-tagged syringe barrels 102 within each respective nest 204 being registered in/with the PLC 410 of conveyor system 407, the PLC 410 may then control operation of the fill and finish machine 409. The fill and finish machine 409 may be operated by PLC 410 to dispense a controlled amount of medicinal fluid into each of RFID-tagged syringe barrels 102, as appropriate for each syringe barrel 102 and as determined by the information contained on the EPC thereof.

Beneficially, embodiments of the invention thus provide a system and method for localizing individual RFID-tagged syringe barrels within a nest, such as may be desired when providing packaged syringe barrels (i.e., packaged in a tub and nest) to a fill and finish manufacturing line. The system and method may read an RFID tag provided on each nest to determine an orientation of that nest (and associated tub), as provided to the manufacturing line, and to identify the nest and the RFID-tagged syringe barrels retained therein. The orientation of a nest can be determined based on the signal responses from the nest RFID tag that are received by a plurality of antennas (e.g., a first antenna and a second antenna) of an RFID reading system - including the strength of the signal responses and/or the order in which the signal responses are received, with the orientation of the nest then being utilized in order to properly localize and locate each RFID-tagged syringe barrel retained within the nest, in order to properly register the RFID-tagged syringe barrels and facilitate filling and finishing thereof. The RFID-tagged syringe barrels and location thereof within a nest may thus be registered regardless of the orientation of the nest (and tub), thus reducing or eliminating potential registration errors on the fill and finish manufacturing line.

Although the present disclosure has been described in detail for the purpose of illustration based on what is currently considered to be the most practical and preferred embodiments or aspects, it is to be understood that such detail is solely for that purpose and that the present disclosure is not limited to the disclosed embodiments or aspects, but, on the contrary, is intended to cover modifications and equivalent arrangements that are within the scope of the appended claims. For example, it is to be understood that the present disclosure contemplates that, to the extent possible, one or more features of any embodiment may be combined with one or more features of any other embodiment.

## Claims

1. A system (400) for localizing RFID-tagged medical injection devices (10) in a nest, the system comprising:
a conveyor system (407) configured to convey a plurality of tubs (202) along a conveying path, each of the plurality of tubs (202) including a nest (204) therein having a nest RFID tag (224) thereon, the nest (204) retaining a plurality of RFID-tagged medical injection devices (10) therein;
a plurality of RFID coupling elements (405) positioned at a reading location along the conveying path, the plurality of RFID coupling elements (405) including at least a first RFID coupling element (405a) provided at a first position of the reading location and a second RFID coupling element (405b) provided at a second position of the reading location;
a RFID reader (404) operably connected with the plurality of RFID coupling elements (405) and configured to perform a reading of the nest RFID tag (224) upon a respective tub (202) passing the plurality of RFID coupling elements (405) at the reading location, wherein reading the nest RFID tag (224) comprises reading a nest identifier from the nest RFID tag (224); the system (400) being **characterized in that** it further comprises:
at least one processor (504) coupled to a memory (506) and configured to:
determine an orientation of the nest (204) based on a reading of the nest RFID tag (224) by at least one of the first RFID coupling element (405a) or the second RFID coupling element (405b), the orientation comprising a first orientation or a second orientation;
query a database (411, 412) using the nest identifier and the determined orientation of the nest, the database (411, 412) including an electronic product code of each of the plurality of RFID-tagged medical injection devices (10) within a respective nest (204) and a location of each of the plurality of RFID-tagged medical injection devices (10) within the respective nest (204) with the nest (204) in each of the first orientation and the second orientation;
register the location of each of the plurality of RFID-tagged medical injection devices (10) within the respective nest (204) based on the query.

2. The system (400) of claim 1, wherein each nest (204) comprises a support plate (208) and a plurality of chimneys (214) extending upwardly from the support plate (208), each of the plurality of chimneys (214) configured to retain a RFID-tagged medical injection device (10) therein, the plurality of chimneys (214) arranged in a plurality of columns (220), with each of the plurality of columns (220) comprising a plurality of seats (222).

3. The system (400) of claim 2, wherein the nest RFID tag (224) is positioned on the support plate (208) of the nest (204).

4. The system (400) of claim 2 or claim 3, wherein registering the location of each of the plurality of RFID-tagged medical injection devices (10) comprises registering the column (220) and seat (222) of each of the plurality of RFID-tagged medical injection devices (10).

5. The system (400) of any of claims 1-4, wherein the first orientation is rotated 180 degrees from the second orientation.

6. The system (400) of any of claims 1-5, wherein the first RFID coupling element (405a) is positioned on a first side of a conveyor (408) of the conveyor system (407) and the second RFID coupling element (405b) is positioned on a second side of the conveyor (408) opposite the first side, and wherein the first RFID coupling element (405a) receives a signal response from the nest RFID tag (224) before the second RFID coupling element (405b) receives a signal response from the nest RFID tag (224) when the nest is in the first orientation, and the second RFID coupling element (405b) receives a signal response from the nest RFID tag (224) before the first RFID coupling element (405a) receives a signal response from the nest RFID tag (224) when the nest (204) is in the second orientation.

7. The system (400) of any of claims 1-5, wherein the first RFID coupling element (405a) is positioned on a first side of a conveyor (408) of the conveyor system and the second RFID coupling element (405b) is positioned on a second side of the conveyor (408) opposite the first side, and wherein the first RFID coupling element (405a) receives a stronger signal response from the nest RFID tag (224) than the second RFID coupling element (405b) when the nest (204) is in the first orientation and the second RFID coupling element (405b) receives a stronger signal response from the nest RFID tag (224) than the first RFID coupling element (405a) when the nest (204) is in the second orientation.

8. The system (400) of any of claims 1-7, further comprising at least one trigger sensor (406) configured to detect when a respective tub (202) is at the reading location;
wherein, for each tub (202) on the conveyor system (407), the at least one sensor (406) is configured to transmit a trigger signal to the RFID reader (404) to cause the RFID reader (404) to read the nest RFID tag (224) of the nest (204) in the respective tub (202) in response to detecting that the tub (202) is at the reading location; and
wherein the RFID reader (404) is configured to read the nest RFID tag (224) on the respective nest (204) in response to receiving the trigger signal from the at least one sensor (406).

9. The system (400) of any of claims 1-8, further comprising a fill and finish machine (409) positioned downstream of the reading location and configured to fill the plurality of RFID-tagged medical injection devices (10) with a fluid, and wherein the at least one processor (504) is further configured to control the fill and finish machine (409) to fill each of the plurality of RFID-tagged medical injection devices (10) with fluid based on the registered location of each of the plurality of RFID-tagged medical injection devices (10) within the respective nest (204) and based on the electronic product code of each of the plurality of RFID-tagged medical injection devices (10).

10. The system (400) of any of claims 1-9, wherein the plurality of RFID-tagged medical injection devices (10) comprise a plurality of syringe barrels (102).

11. A method for localizing RFID-tagged medical injection devices (10) in a nest (204), the method comprising:
conveying each of a plurality of tubs (202), via a conveyor system (407), along a conveying path, each of the plurality of tubs (202) including a nest (204) therein having a nest RFID tag (224) thereon, the nest retaining a plurality of RFID-tagged medical injection devices therein;
providing a RFID reading system at a position along the conveying path, the RFID reading system comprising:
a plurality of RFID coupling elements (405) positioned at a reading location along the conveying path, the plurality of RFID coupling elements (405) including at least a first RFID coupling element (405a) provided at a first position of the reading location and a second RFID coupling element (405b) provided at a second position of the reading location; and
a RFID reader (404) operably connected with the plurality of RFID coupling elements (405);
performing a reading of each of nest RFID tag (224) via the RFID reading system, as the respective tubs (202) pass the reading location; and
based on the reading of each of the plurality of nest RFID tags (224), determining, via at least one processor (504), a location of each of the plurality of RFID-tagged medical injection devices (10) within a respective nest (204);
wherein, in determining the location of each of the plurality of RFID-tagged medical injection devices (10), the method comprises:
determining an orientation of the nest (204) based on a reading of the nest RFID tag (224) by at least one of the first RFID coupling element (405a) or the second RFID coupling element (405b), the orientation comprising a first orientation or a second orientation;
querying a database (411, 412) using the nest identifier and the determined orientation of the nest, the database (411, 412) including an electronic product code of each of the plurality of RFID-tagged medical injection devices (10) within a respective nest (204) and a location of each of the plurality of RFID-tagged medical injection devices (10) within a respective nest (204) with the nest (204) in each of the first orientation and the second orientation; and
registering the location of each of the plurality of RFID-tagged medical injection devices (10) within the respective nest (204) based on the query.

12. The method of claim 11, wherein each nest (204) comprises a plurality of chimneys (214) each configured to retain a RFID-tagged medical injection device (10) therein, the plurality of chimneys (214) arranged in a plurality of columns (220), with each of the plurality of columns (220) comprising a plurality of seats (222), and wherein registering the location of each of the plurality of RFID-tagged medical injection devices (10) comprises registering the column (220) and seat (222) of each of the plurality of RFID-tagged medical injection devices (10).

13. The method of claim 11 or claim 12, wherein performing a reading of each of nest RFID tag (224) via the RFID reading system comprises receiving a signal response from the nest RFID tag (224) at the first RFID coupling element (405a) before receiving the signal response at the second RFID coupling element (405b) when the nest (204) is in the first orientation or receiving a signal response from the nest RFID tag (224) at the second RFID coupling element (405b) before receiving the signal response at the first RFID coupling element (405a) when the nest (204) is in the second orientation.

14. The method of claim 11 or claim 12, wherein performing a reading of each of nest RFID tag (224) via the RFID reading system comprises receiving a signal response from the nest RFID tag (224) at the first RFID coupling element (405a) that is stronger than a signal response received at the second RFID coupling element (405b) when the nest (204) is in the first orientation or receiving a signal response from the nest RFID tag (224) at the second RFID coupling element (405b) that is stronger than a signal response received at the first RFID coupling element (405a) when the nest is in the second orientation.

15. The method of any of claims 11-14, further comprising:
detecting when a respective tub (202) is at the reading location using at least one trigger sensor (406); and
in response to detecting that the tub (202) is at the reading location, transmitting a trigger signal from the at least one trigger sensor (406) to the RFID reader (404) to cause the RFID reader (404) to read the nest RFID tag (224) of a respective nest (204) contained within the tub (202).

16. The method of any of claims 11-15, further comprising controlling a fill and finish machine (409) to fill each of the plurality of RFID-tagged medical injection devices (10) retained in a respective nest (204) with fluid based on the registered location of each of the plurality of RFID-tagged medical injection devices (10) within the nest (204) and based on the electronic product code of each of the plurality of RFID-tagged medical injection devices (10) retained in the nest (204).

17. The method of any of claims 11-16, wherein the first orientation is rotated 180 degrees from the second orientation.

## Patentansprüche

1. System (400) zum Lokalisieren von RFID-gekennzeichneten medizinischen Injektionsvorrichtungen (10) in einem Nest, das System umfassend:
ein Fördersystem (407), das so konfiguriert ist, dass es eine Vielzahl von Wannen (202) entlang eines Förderpfads fördert, wobei jede der Vielzahl von Wannen (202) ein Nest (204) darin aufweist, das ein Nest-RFID-Tag (224) darauf aufweist, wobei das Nest (204) eine Vielzahl von RFID-gekennzeichneten medizinischen Injektionsvorrichtungen (10) darin aufhält;
eine Vielzahl von RFID-Kopplungselementen (405), die an einem Lesestandort entlang des Förderwegs positioniert sind, wobei die Vielzahl von RFID-Kopplungselementen (405) mindestens ein erstes RFID-Kopplungselement (405a), das an einer ersten Position des Lesestandorts bereitgestellt ist, und eines zweiten RFID-Kopplungselements (405b), das an einer zweiten Position des Lesestandorts bereitgestellt ist, enthalten;
ein RFID-Lesegerät (404), das betriebsbereit mit der Vielzahl von RFID-Kopplungselementen (405) verbunden ist und so konfiguriert ist, dass es das Lesen des Nest-RFID-Tags (224) auf einer jeweiligen Wanne (202) durchführt, der die Vielzahl von RFID-Kopplungselementen (405) an dem Lesestandort passiert, wobei das Lesen des Nest-RFID-Tags (224) das Lesen eines Nest-Identifikators vom Nest-RFID-Tag (224) umfasst; wobei das System (400) **dadurch gekennzeichnet ist, dass** es ferner Folgendes umfasst:
mindestens einen Prozessor (504), der mit einem Speicher (506) gekoppelt und zu Folgendem konfiguriert ist:
Bestimmen einer Ausrichtung des Nests (204) basierend auf einem Lesen des Nest-RFID-Tags (224) durch mindestens eines der ersten RFID-Kopplungselemente (405a) oder des zweiten RFID-Kopplungselements (405b), wobei die Ausrichtung eine erste Ausrichtung oder eine zweite Ausrichtung umfasst;
Abfragen einer Datenbank (411, 412) unter Verwendung der Nest-Kennung und der bestimmten Ausrichtung des Nests, wobei die Datenbank (411, 412) einen elektronischen Produktcode jeder der Vielzahl von von RFID-gekennzeichneten medizinischen Injektionsvorrichtungen (10) innerhalb eines jeweiligen Nests (204) und einen Standort jeder der Vielzahl von von RFID-gekennzeichneten medizinischen Injektionsvorrichtungen (10) innerhalb des jeweiligen Nests (204) mit dem Nest (204) in jeder der ersten Ausrichtung und der zweiten Ausrichtung enthält;
Registrieren des Standorts jeder der Vielzahl von von RFID-gekennzeichneten medizinischen Injektionsvorrichtungen (10) innerhalb des jeweiligen Nests (204) basierend auf der Abfrage.

2. System (400) nach Anspruch 1, wobei jedes Nest (204) eine Stützplatte (208) und eine Vielzahl von Schächten (214) umfasst, die sich nach oben von der Stützplatte (208) erstrecken, wobei jeder der Vielzahl von Schächten (214) so konfiguriert ist, dass er eine von RFID-gekennzeichnete medizinische Injektionsvorrichtung (10) darin aufnimmt, wobei die die Vielzahl von Schächten (214) in einer Vielzahl von Säulen (220) angeordnet sind, wobei jeder der Vielzahl von Säulen (220) eine Vielzahl von Sitzen (222) umfasst.

3. System (400) nach Anspruch 2, wobei das Nest-RFID-Tag (224) auf der Stützplatte (208) des Nests (204) positioniert ist.

4. System (400) nach Anspruch 2 oder Anspruch 3, wobei das Registrieren des Standorts jeder der Vielzahl von von RFID-gekennzeichneten medizinischen Injektionsvorrichtungen (10) das Registrieren der Säule (220) und des Sitzes (222) jeder der Vielzahl von RFID-gekennzeichneten medizinischen Injektionsvorrichtungen (10) umfasst.

5. System (400) nach einem der Ansprüche 1 bis 4, wobei die erste Ausrichtung um 180 Grad von der zweiten Ausrichtung gedreht wird.

6. System (400) nach einem der Ansprüche 1 bis 5, wobei das erste RFID-Kopplungselement (405a) auf einer ersten Seite eines Förderers (408) des Fördersystems (407) und das zweite RFID-Kopplungselement (405b) auf einer zweiten Seite des Förderers (408) gegenüber der ersten Seite positioniert ist, und wobei das erste RFID-Kopplungselement (405a) eine Signalantwort von dem Nest-RFID-Tag (224) empfängt, bevor das zweite RFID-Kopplungselement (405b) eine Signalantwort von dem Nest-RFID-Tag (224) empfängt, wenn sich das Nest in der ersten Ausrichtung befindet und das zweite RFID-Kopplungselement (405b) eine Signalantwort von dem Nest-RFID-Tag (224) empfängt, bevor das erste RFID-Kopplungselement (405a) eine Signalantwort von dem Nest-RFID-Tag (224) empfängt, wenn sich das Nest (204) in der zweiten Ausrichtung befindet.

7. System (400) nach einem der Ansprüche 1 bis 5, wobei das erste RFID-Kopplungselement (405a) auf einer ersten Seite eines Förderers (408) des Fördersystems positioniert ist und das zweite RFID-Kopplungselement (405b) auf einer zweiten Seite des Förderers (408) gegenüber der ersten Seite positioniert ist, und wobei das erste RFID-Kopplungselement (405a) eine stärkere Signalantwort von dem Nest-RFID-Tag (224) empfängt als das zweite RFID-Kopplungselement (405b), wenn das Nest (204) in der ersten Ausrichtung ist und das zweite RFID-Kopplungselement (405b) eine stärkere Signalantwort von dem Nest-RFID-Tag (224) empfängt als das erste RFID-Kopplungselement (405a), wenn sich das Nest (204) in der zweiten Ausrichtung befindet.

8. System (400) nach einem der Ansprüche 1 bis 7, ferner umfassend mindestens einen Auslösesensor (406), der so konfiguriert ist, dass er erkennt, wenn sich eine jeweilige Wanne (202) an dem Lesestandort befindet;
wobei für jede Wanne (202) auf dem Fördersystem (407) der mindestens eine Sensor (406) so konfiguriert ist, dass er ein Triggersignal an den RFID-Leser (404) sendet, um den RFID-Leser (404) dazu zu veranlassen, das Nest-RFID-Tag (224) des Nests (204) in der jeweiligen Wanne (202) als Antwort darauf zu lesen, dass sich die Wanne (202) an dem Lesestandort befindet; und
wobei der RFID-Leser (404) so konfiguriert ist, dass er das Nest-RFID-Tag (224) auf dem jeweiligen Nest (204) als Antwort auf den Empfang des Triggersignals von dem mindestens einen Sensor (406) liest.

9. System (400) nach einem der Ansprüche 1 bis 8, ferner umfassend eine Füll- und Veredelungsmaschine (409), die nach dem Lesestandort positioniert und so konfiguriert ist, dass sie die Vielzahl von von RFID-gekennzeichneten medizinischen Injektionsvorrichtungen (10) mit einem Fluid füllt, und wobei der mindestens eine Prozessor (504) ferner so konfiguriert ist, dass er die Füll- und Veredelungsmaschine (409) steuert, um jede der Vielzahl von von RFID-gekennzeichneten medizinischen Injektionsvorrichtungen (10) mit Fluid zu füllen, basierend auf dem registrierten Standort jeder der Vielzahl von von RFID-gekennzeichneten medizinischen Injektionsvorrichtungen (10) innerhalb des jeweiligen Nests (204) und basierend auf dem elektronischen Produktcode jeder der Vielzahl von von RFID-gekennzeichneten medizinischen Injektionsvorrichtungen (10).

10. System (400) nach einem der Ansprüche 1 bis 9, wobei die Vielzahl von RFID-gekennzeichneten medizinischen Injektionsvorrichtungen (10) eine Vielzahl von Spritzenfässer (102) umfassen.

11. Verfahren zum Lokalisieren von RFID-gekennzeichneten medizinischen Injektionsvorrichtungen (10) in einem Nest (204), wobei das Verfahren Folgendes umfasst:
Befördern jeder Vielzahl von Wannen (202) über ein Fördersystem (407) entlang eines Förderpfads, wobei jede der Vielzahl der Wannen (202) ein Nest (204) enthält, das ein Nest-RFID-Tag (224) darauf aufweist, wobei das Nest eine Vielzahl von medizinischen Injektionsvorrichtungen mit RFID-Tag darin aufnimmt;
Bereitstellen eines RFID-Lesesystems an einer Position entlang des Förderwegs, wobei das RFID-Lesesystem Folgendes umfasst:
eine Vielzahl von RFID-Kopplungselementen (405), die an einem Lesestandort entlang des Förderwegs positioniert sind, wobei die Vielzahl der RFID-Kopplungselemente (405) mindestens ein erstes RFID-Kopplungselement (405a), das an einer ersten Position des Lesestandorts bereitgestellt ist, und ein zweites RFID-Kopplungselement (405b), das an einer zweiten Position des Lesestandorts bereitgestellt ist, enthält; und
einen RFID-Leser (404), der betriebsbereit mit der Vielzahl von RFID-Kopplungselementen (405) verbunden ist;
Auslesen jedes der Nest-RFID-Tags (224) über das RFID-Lesesystem, während die jeweiligen Wannen (202) den Lesestandort passieren; und
basierend auf dem Lesen jedes der Vielzahl von Nest-RFID-Tags (224), Bestimmen, über mindestens einen Prozessor (504), eines Standorts jeder der Vielzahl von von RFID-gekennzeichneten medizinischen Injektionsvorrichtungen (10) innerhalb eines jeweiligen Nests (204);
wobei das Verfahren bei dem Bestimmen des Standorts jeder der Vielzahl von von RFID-gekennzeichneten medizinischen Injektionsvorrichtungen (10) Folgendes umfasst:
Bestimmen einer Ausrichtung des Nests (204) basierend auf einem Lesen des Nest-RFID-Tags (224) durch mindestens eines des ersten RFID-Kopplungselements (405a) oder des zweiten RFID-Kopplungselements (405b), wobei die Ausrichtung eine erste Ausrichtung oder eine zweite Ausrichtung umfasst;
Abfragen einer Datenbank (411, 412) unter Verwendung der Nest-Kennung und der bestimmten Ausrichtung des Nests, wobei die Datenbank (411, 412) einen elektronischen Produktcode jeder der Vielzahl von von RFID-gekennzeichneten medizinischen Injektionsvorrichtungen (10) innerhalb eines jeweiligen Nests (204) und einen Standort jeder der Vielzahl von von RFID-gekennzeichneten medizinischen Injektionsvorrichtungen (10) innerhalb eines jeweiligen Nests (204) mit dem Nest (204) in jeder der ersten Ausrichtung und der zweiten Ausrichtung enthält; und
Registrieren des Standorts jeder der Vielzahl von von RFID-gekennzeichneten medizinischen Injektionsvorrichtungen (10) innerhalb des jeweiligen Nests (204) basierend auf der Abfrage.

12. Verfahren nach Anspruch 11, wobei jedes Nest (204) eine Vielzahl von Schächten (214) umfasst, die jeweils so konfiguriert sind, dass sie eine von RFID-gekennzeichnete medizinische Injektionsvorrichtung (10) darin zurückhalten, wobei die Vielzahl von Schächten (214) in einer Vielzahl von Säulen (220) angeordnet sind, wobei jede der Vielzahl von Säulen (220) eine Vielzahl von Sitzen (222) umfasst, und wobei das Registrieren des Standorts jedes der Vielzahl von ovn RFID-gekennzeichneten medizinischen Injektionsvorrichtungen (10) das Registrieren der Säule (220) und des Sitzes (222) jeder der Vielzahl von von RFID-gekennzeichneten medizinischen Injektionsvorrichtungen (10) umfasst.

13. Verfahren nach Anspruch 11 oder Anspruch 12, wobei das Auslesen jedes Nest-RFID-Tags (224) über das RFID-Lesesystem den Empfang einer Signalantwort vom Nest-RFID-Tag (224) am ersten RFID-Kopplungselement (405a) umfasst, bevor die Signalantwort am zweiten RFID-Kopplungselement (405b) empfangen wird, wenn sich das Nest (204) in der ersten Ausrichtung befindet, oder eine Signalantwort vom Nest-RFID-Tag (224) am zweiten RFID-Kopplungselement (405b) empfangen wird bevor sie die Signalantwort am ersten RFID-Kopplungselement (405a) empfängt, wenn sich das Nest (204) in der zweiten Ausrichtung befindet.

14. Verfahren nach Anspruch 11 oder Anspruch 12, wobei das Auslesen jedes Nest-RFID-Tags (224) über das RFID-Lesesystem das Empfangen einer Signalantwort vom Nest-RFID-Tag (224) am ersten RFID-Kopplungselement (405a) umfasst, die stärker ist als eine Signalantwort, die am zweiten RFID-Kopplungselement (405b) empfangen wird, wenn sich das Nest (204) in der ersten Ausrichtung befindet, oder das Empfangen einer Signalantwort vom Nest-RFID-Tag (224) am zweiten RFID-Kopplungselement (405b), die stärker ist als eine an dem ersten RFID-Kopplungselement (405a) empfangene Signalantwort, wenn sich das Nest in der zweiten Ausrichtung befindet.

15. Verfahren nach einem der Ansprüche 11 bis 14, ferner umfassend:
Erkennen, wann sich eine jeweilige Wanne (202) an dem Lesestandort befindet, mithilfe von mindestens einem Auslösesensor (406); und
als Antwort auf das Erkennen, dass sich die Wanne (202) an dem Lesestandort befindet, das Senden eines Triggersignals von dem mindestens einen Triggersensor (406) an den RFID-Leser (404), um den RFID-Leser (404) dazu zu veranlassen, das Nest-RFID-Tag (224) eines jeweiligen Nests (204) zu lesen, das sich in der Wanne (202) befindet.

16. Verfahren nach einem der Ansprüche 11 bis 15, ferner umfassend das Steuern einer Füll- und Endbearbeitungsmaschine (409) zum Befüllen jeder der Vielzahl von von RFID-gekennzeichneten medizinischen Injektionsvorrichtungen (10), die in einem jeweiligen Nest (204) zurückgehalten werden, mit Fluid, basierend auf dem registrierten Standort jeder der Vielzahl von von RFID-gekennzeichneten medizinischen Injektionsvorrichtungen (10) innerhalb des Nests (204) und basierend auf dem elektronischen Produktcode jeder der Vielzahl von von RFID-gekennzeichneten medizinischen Injektionsvorrichtungen (10), die in dem Nest (204) zurückgehalten werden.

17. Verfahren nach einem der Ansprüche 11 bis 16, wobei die erste Ausrichtung um 180 Grad von der zweiten Ausrichtung gedreht wird.

## Revendications

1. Système (400) pour localiser des dispositifs médicaux d'injection à étiquette RFID (10) dans un nid, le système comprenant :
un système de transport (407) configuré pour transporter une pluralité de bacs (202) le long d'un chemin de transport, chacun de la pluralité de bacs (202) comprenant un nid (204) dedans ayant une étiquette RFID de nid (224) sur celui-ci, le nid (204) retenant une pluralité de dispositifs médicaux d'injection à étiquette RFID (10) dedans ;
une pluralité d'éléments de couplage RFID (405) positionnés à un emplacement de lecture le long du chemin de transport, la pluralité d'éléments de couplage RFID (405) comprenant au moins un premier élément de couplage RFID (405a) prévu à une première position de l'emplacement de lecture et un second élément de couplage RFID (405b) prévu à une seconde position de l'emplacement de lecture ;
un lecteur RFID (404) relié de manière fonctionnelle à la pluralité d'éléments de couplage RFID (405) et configuré pour réaliser une lecture de l'étiquette RFID de nid (224) lors du passage d'un bac respectif (202) devant la pluralité d'éléments de couplage RFID (405) à l'emplacement de lecture, où la lecture de l'étiquette RFID de nid (224) comprend la lecture d'un identifiant de nid à partir de l'étiquette RFID de nid (224) ; le système (400) étant **caractérisé en ce qu'**il comprend en outre :
au moins un processeur (504) couplé à une mémoire (506) et configuré pour :
déterminer une orientation du nid (204) sur la base d'une lecture de l'étiquette RFID de nid (224) par au moins l'un parmi le premier élément de couplage RFID (405a) ou le second élément de couplage RFID (405b), l'orientation comprenant une première orientation ou une seconde orientation ;
interroger une base de données (411, 412) en utilisant l'identifiant de nid et l'orientation déterminée du nid, la base de données (411, 412) comprenant un code de produit électronique de chacun de la pluralité de dispositifs médicaux d'injection à étiquette RFID (10) dans un nid respectif (204) et un emplacement de chacun de la pluralité de dispositifs médicaux d'injection à étiquette RFID (10) dans le nid respectif (204) avec le nid (204) dans chacune de la première orientation et de la seconde orientation
enregistrer l'emplacement de chacun de la pluralité de dispositifs médicaux d'injection à étiquette RFID (10) dans le nid respectif (204) sur la base de l'interrogation.

2. Système (400) selon la revendication 1, dans lequel chaque nid (204) comprend une plaque de support (208) et une pluralité de cheminées (214) s'étendant vers le haut depuis la plaque de support (208), chacune de la pluralité de cheminées (214) étant configurée pour retenir un dispositif médical d'injection à étiquette RFID (10) dedans, la pluralité de cheminées (214) étant agencées en une pluralité de colonnes (220), chacune de la pluralité de colonnes (220) comprenant une pluralité de logements (222).

3. Système (400) selon la revendication 2, dans lequel l'étiquette RFID de nid (224) est positionnée sur la plaque de support (208) du nid (204).

4. Système (400) selon la revendication 2 ou 3, dans lequel l'enregistrement de l'emplacement de chacun de la pluralité de dispositifs médicaux d'injection à étiquette RFID (10) comprend l'enregistrement de la colonne (220) et du logement (222) de chacun de la pluralité de dispositifs médicaux d'injection à étiquette RFID (10).

5. Système (400) selon l'une des revendications 1 à 4, dans lequel la première orientation tourne de 180 degrés par rapport à la seconde orientation.

6. Système (400) selon l'une des revendications 1 à 5, dans lequel le premier élément de couplage RFID (405a) est positionné sur un premier côté d'un transporteur (408) du système de transport (407) et le second élément de couplage RFID (405b) est positionné sur un second côté du transporteur (408) opposé au premier côté, et dans lequel le premier élément de couplage RFID (405a) reçoit une réponse de signal de l'étiquette RFID de nid (224) avant que le second élément de couplage RFID (405b) ne reçoive une réponse de signal de l'étiquette RFID de nid (224) lorsque le nid est dans la première orientation, et le second élément de couplage RFID (405b) reçoit une réponse de signal de l'étiquette RFID de nid (224) avant que le premier élément de couplage RFID (405a) ne reçoive une réponse de signal de l'étiquette RFID de nid (224) lorsque le nid (204) est dans la seconde orientation.

7. Système (400) selon l'une des revendications 1 à 5, dans lequel le premier élément de couplage RFID (405a) est positionné sur un premier côté d'un transporteur (408) du système de transport et le second élément de couplage RFID (405b) est positionné sur un second côté du transporteur (408) opposé au premier côté, et dans lequel le premier élément de couplage RFID (405a) reçoit une réponse de signal plus forte de l'étiquette RFID de nid (224) que le second élément de couplage RFID (405b) lorsque le nid (204) est dans la première orientation et le second élément de couplage RFID (405b) reçoit une réponse de signal plus forte de l'étiquette RFID de nid (224) que le premier élément de couplage RFID (405a) lorsque le nid (204) est dans la seconde orientation.

8. Système (400) selon l'une des revendications 1 à 7, comprenant en outre au moins un capteur de déclenchement (406) configuré pour détecter le moment où un bac respectif (202) se trouve à l'emplacement de lecture ;
dans lequel, pour chaque bac (202) sur le système de transport (407), l'au moins un capteur (406) est configuré pour transmettre un signal de déclenchement au lecteur RFID (404) afin d'amener le lecteur RFID (404) à lire l'étiquette RFID de nid (224) du nid (204) dans le bac respectif (202) en réponse à la détection du fait que le bac (202) se trouve à l'emplacement de lecture ; et
dans lequel le lecteur RFID (404) est configuré pour lire l'étiquette RFID de nid (224) sur le nid respectif (204) en réponse à la réception du signal de déclenchement de l'au moins un capteur (406).

9. Système (400) selon l'une des revendications 1 à 8, comprenant en outre une machine de remplissage et de finition (409) positionnée en aval de l'emplacement de lecture et configurée pour remplir la pluralité de dispositifs médicaux d'injection à étiquette RFID (10) avec un fluide, et où l'au moins un processeur (504) est en outre configuré pour commander la machine de remplissage et de finition (409) afin de remplir chacun de la pluralité de dispositifs médicaux d'injection à étiquette RFID (10) avec un fluide sur la base de l'emplacement enregistré de chacun de la pluralité de dispositifs médicaux d'injection à étiquette RFID (10) dans le nid respectif (204) et sur la base du code de produit électronique de chacun de la pluralité de dispositifs médicaux d'injection à étiquette RFID (10).

10. Système (400) selon l'une des revendications 1 à 9, dans lequel la pluralité de dispositifs médicaux d'injection à étiquette RFID (10) comprend une pluralité de cylindres de seringues (102).

11. Procédé de localisation de dispositifs médicaux d'injection à étiquette RFID (10) dans un nid (204), le procédé comprenant :
le transport de chacun d'une pluralité de bacs (202), via un système de transport (407), le long d'un chemin de transport, chacun de la pluralité de bacs (202) comprenant un nid (204) dedans ayant une étiquette RFID de nid (224) sur celui-ci, le nid retenant une pluralité de dispositifs médicaux d'injection à étiquette RFID dedans ;
la fourniture d'un système de lecture RFID à une position le long du chemin de transport, le système de lecture RFID comprenant :
une pluralité d'éléments de couplage RFID (405) positionnés à un emplacement de lecture le long du chemin de transport, la pluralité d'éléments de couplage RFID (405) comprenant au moins un premier élément de couplage RFID (405a) prévu à une première position de l'emplacement de lecture et un second élément de couplage RFID (405b) prévu à une seconde position de l'emplacement de lecture ; et
un lecteur RFID (404) relié de manière fonctionnelle à la pluralité d'éléments de couplage RFID (405) ;
la réalisation d'une lecture de chacune des étiquettes RFID de nid (224) via le système de lecture RFID, à mesure que les bacs respectifs (202) passent devant l'emplacement de lecture ; et
sur la base de la lecture de chacune de la pluralité d'étiquettes RFID de nid (224), la détermination, via au moins un processeur (504), d'un emplacement de chacun de la pluralité de dispositifs médicaux d'injection à étiquette RFID (10) dans un nid respectif (204) ;
dans lequel, lors de la détermination de l'emplacement de chacun de la pluralité de dispositifs médicaux d'injection à étiquette RFID (10), le procédé comprend :
la détermination d'une orientation du nid (204) sur la base d'une lecture de l'étiquette RFID de nid (224) par au moins l'un parmi le premier élément de couplage RFID (405a) ou le second élément de couplage RFID (405b), l'orientation comprenant une première orientation ou une seconde orientation ;
l'interrogation d'une base de données (411, 412) en utilisant l'identifiant de nid et l'orientation déterminée du nid, la base de données (411, 412) comprenant un code de produit électronique de chacun de la pluralité de dispositifs médicaux d'injection à étiquette RFID (10) dans un nid respectif (204) et un emplacement de chacun de la pluralité de dispositifs médicaux d'injection à étiquette RFID (10) dans un nid respectif (204) avec le nid (204) dans chacune de la première orientation et de la seconde orientation ; et
l'enregistrement de l'emplacement de chacun de la pluralité de dispositifs médicaux d'injection à étiquette RFID (10) dans le nid respectif (204) sur la base de l'interrogation.

12. Procédé selon la revendication 11, dans lequel chaque nid (204) comprend une pluralité de cheminées (214) configurées chacune pour retenir un dispositif médical d'injection à étiquette RFID (10) dedans, la pluralité de cheminées (214) étant agencées en une pluralité de colonnes (220), chacune de la pluralité de colonnes (220) comprenant une pluralité de logements (222), et dans lequel l'enregistrement de l'emplacement de chacun de la pluralité de dispositifs médicaux d'injection à étiquette RFID (10) comprend l'enregistrement de la colonne (220) et du logement (222) de chacun de la pluralité de dispositifs médicaux d'injection à étiquette RFID (10).

13. Procédé selon la revendication 11 ou 12, dans lequel la réalisation d'une lecture de chacune des étiquettes RFID de nid (224) via le système de lecture RFID comprend la réception d'une réponse de signal de l'étiquette RFID de nid (224) au niveau du premier élément de couplage RFID (405a) avant la réception de la réponse de signal au niveau du second élément de couplage RFID (405b) lorsque le nid (204) est dans la première orientation ou la réception d'une réponse de signal de l'étiquette RFID de nid (224) au niveau du second élément de couplage RFID (405b) avant la réception de la réponse de signal au niveau du premier élément de couplage RFID (405a) lorsque le nid (204) est dans la seconde orientation.

14. Procédé selon la revendication 11 ou 12, dans lequel la réalisation d'une lecture de chacune des étiquettes RFID de nid (224) via le système de lecture RFID comprend la réception d'une réponse de signal de l'étiquette RFID de nid (224) au niveau du premier élément de couplage RFID (405a) qui est plus forte qu'une réponse de signal reçue au niveau du second élément de couplage RFID (405b) lorsque le nid (204) est dans la première orientation ou la réception d'une réponse de signal de l'étiquette RFID de nid (224) au niveau du second élément de couplage RFID (405b) qui est plus forte qu'une réponse de signal reçue au niveau du premier élément de couplage RFID (405a) lorsque le nid est dans la seconde orientation.

15. Procédé selon l'une des revendications 11 à 14, comprenant en outre :
la détection du moment où un bac respectif (202) se trouve à l'emplacement de lecture en utilisant au moins un capteur de déclenchement (406) ; et
en réponse à la détection du fait que le bac (202) se trouve à l'emplacement de lecture, la transmission d'un signal de déclenchement de l'au moins un capteur de déclenchement (406) vers le lecteur RFID (404) afin d'amener le lecteur RFID (404) à lire l'étiquette RFID de nid (224) d'un nid respectif (204) contenu dans le bac (202).

16. Procédé selon l'une des revendications 11 à 15, comprenant en outre la commande d'une machine de remplissage et de finition (409) pour remplir chacun de la pluralité de dispositifs médicaux d'injection à étiquette RFID (10) retenus dans un nid respectif (204) avec un fluide sur la base de l'emplacement enregistré de chacun de la pluralité de dispositifs médicaux d'injection à étiquette RFID (10) dans le nid (204) et sur la base du code de produit électronique de chacun de la pluralité de dispositifs médicaux d'injection à étiquette RFID (10) retenus dans le nid (204).

17. Procédé selon l'une des revendications 11 à 16, dans lequel la première orientation tourne de 180 degrés par rapport à la seconde orientation.
